# EUROPEAN PATENT SPECIFICATION

(11) **EP 4 157 845 B1**
(45) Date of publication and mention of the grant of the patent: **13.08.2025**
(21) Application number: 21729021.2
(22) Date of filing: 25.05.2021
(51) Int. Cl.: C07D 487/04, A61P 1/04, A61P 3/10, A61P 5/14, A61P 7/00, A61P 9/00, A61P 11/00, A61P 13/12, A61K 31/5377

(54) **PYRROLO[2,3-D]PYRIMIDINE DERIVATIVES**
PYRROLO[2,3-D]PYRIMIDINDERIVATE
DÉRIVÉS DE PYRROLO [2,3-D] PYRIMIDINE

(30) Priority: 28.05.2020 US 202062704796 P; 10.06.2020 US 202063037366 P
(43) Date of publication of application: 05.04.2023
(73) Proprietor: Pfizer Inc., New York, NY 10001-2192 (US)
(72) Inventor: DOWTY, Martin Eugene, Cambridge, Massachusetts 02139 (US); MALHOTRA, Bimal Kumar, New York, New York 10017 (US); SAMARDJIEV, Ivan Jordan, Groton, Connecticut 06340 (US); SAMAS, Brian Matthew, Groton, Connecticut 06340 (US); STROHBACH, Joseph Walter, Cambridge, Massachusetts 02139 (US)
(74) Representative: Pfizer
(86) International application number: PCT/IB2021/054516
(87) International publication number: WO 2021/240356

(56) References cited:
- US-A1- 2014 243 312
- US-A1- 2016 045 508
- STEPHEN T. WROBLESKI ET AL: "Highly Selective Inhibition of Tyrosine Kinase 2 (TYK2) for the Treatment of Autoimmune Diseases: Discovery of the Allosteric Inhibitor BMS-986165", JOURNAL OF MEDICINAL CHEMISTRY, 18 July 2019 (2019-07-18), XP055629848, ISSN: 0022-2623, DOI: 10.1021/acs.jmedchem.9b00444
- MICHAEL L. VAZQUEZ ET AL: "Identification of N -{ cis -3-[Methyl(7 H -pyrrolo[2,3- d ]pyrimidin-4-yl)amino]cyclobutyl}propane-1-sulfonamide (PF-04965842): A Selective JAK1 Clinical Candidate for the Treatment of Autoimmune Diseases", JOURNAL OF MEDICINAL CHEMISTRY, vol. 61, no. 3, 23 January 2018 (2018-01-23), pages 1130 - 1152, XP055629803, ISSN: 0022-2623, DOI: 10.1021/acs.jmedchem.7b01598

## Description

### FIELD OF THE INVENTION

The present invention relates to pyrrolo[2,3-d]pyrimidine compounds and isolated forms of such compounds. The invention also relates to compositions containing said compounds and said compounds for use in methods for treating and preventing conditions mediated by JAK1.

### BACKGROUND OF THE INVENTION

Protein kinases are families of enzymes that catalyze the phosphorylation of specific residues in proteins, broadly classified into tyrosine and serine/threonine kinases. Inappropriate kinase activity, arising from mutation, over-expression, or inappropriate regulation, dysregulation or de-regulation, as well as over- or under-production of growth factors or cytokines has been implicated in many diseases, including but not limited to cancer, cardiovascular diseases, allergies, asthma and other respiratory diseases, autoimmune diseases, inflammatory diseases, bone diseases, metabolic disorders, and neurological and neurodegenerative disorders such as Alzheimer's disease. Inappropriate kinase activity triggers a variety of biological cellular responses relating to cell growth, cell differentiation, survival, apoptosis, mitogenesis, cell cycle control, and cell mobility implicated in the aforementioned and related diseases.

Thus, protein kinases have emerged as an important class of enzymes as targets for therapeutic intervention. In particular, the JAK family of cellular protein tyrosine kinases (JAK1, JAK2, JAK3, and Tyk2) play a central role in cytokine signaling (Kisseleva et al., Gene, 2002, 285, 1; Yamaoka et al. Genome Biology 2004, 5, 253)). Upon binding to their receptors, cytokines activate JAK which then phosphorylate the cytokine receptor, thereby creating docking sites for signaling molecules, notably, members of the signal transducer and activator of transcription (STAT) family that ultimately lead to gene expression. Numerous cytokines are known to activate the JAK family. These cytokines include, the IFN family (IFN-alpha, IFN-beta, IFN-omega, Limitin, IFN-gamma, IL-10, IL-19, IL-20, IL-22), the gp130 family (IL-6, IL-11, OSM, LIF, CNTF, NNT-1/BSF-3, G-CSF, CT-1, Leptin, IL-12, IL-23), gamma C family (IL-2, IL-7, TSLP, IL-9, IL-15, IL-21, IL-4, IL-13), IL-3 family (IL-3, IL-5, GM-CSF), single chain family (EPO, GH, PRL, TPO), receptor tyrosine kinases (EGF, PDGF, CSF-1, HGF), and G-protein coupled receptors (AT1).

There remains a need for new compounds that effectively and selectively inhibit specific JAK enzymes, and JAK1 in particular, vs. JAK2. JAK1 is a member of the Janus family of protein kinases composed of JAK1, JAK2, JAK3 and TYK2. JAK1 is expressed to various levels in all tissues. Many cytokine receptors signal through pairs of JAK kinases in the following combinations: JAK1/JAK2, JAK1/JAK3, JAK1/TYK2, JAK2/TYK2 or JAK2/JAK2. JAK1 is the most broadly paired JAK kinase in this context and is required for signaling by γ-common (IL-2Rγ) cytokine receptors, IL-6 receptor family, Type I, II and III receptor families and IL-10 receptor family. Animal studies have shown that JAK1 is required for the development, function and homeostasis of the immune system. Modulation of immune activity through inhibition of JAK1 kinase activity can prove useful in the treatment of various immune disorders (Murray, P.J. J. Immunol., 178, 2623-2629 (2007); Kisseleva, T., et al., Gene, 285, 1-24 (2002); O'Shea, J. J., et al., Cell, 109, (suppl.) S121-S131 (2002)) while avoiding JAK2 dependent erythropoietin (EPO) and thrombopoietin (TPO) signaling (Neubauer H., et al., Cell, 93(3), 397-409 (1998); Parganas E., et al., Cell, 93(3), 385-95 (1998)).

There are substantial needs for safe and efficacious agents to control disorders related to JAK, such as atopic dermatitis, both in human and animals. The market for treating atopic dermatitis is currently dominated by corticosteroids, which cause distressing and undesirable side effects. Antihistamines are also used, but are poorly effective. New compounds which are JAK inhibitors with selective efficacy against JAK1 are needed which provide an alternative to steroid usage and promote resolution of chronic pruritus and inflammation that would either persist in atopic dermatitis or slowly regress following removal of allergen or causative agent.

Recently identified N-((1S,3S)-3-(Methyl(7H-pyrrolo[2,3-d]pyrimidin-4-yl)amino)cyclobutyl)propane-1-sulfonamide, generically known as abrocitinib, is described in commonly assigned US9,035,074, and has the chemical formula C₁₄H₂₁N₅O₂S and the following structural formula: 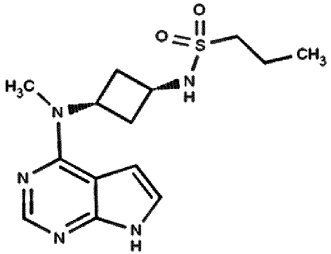 It is known that abrocitinib is a highly promising inhibitor of JAK, and is useful therapeutically for the treatment of a variety of disorders including atopic dermatitis. Novel metabolites of abrocitinib have now been discovered by the present inventors that are highly active as inhibitors of JAK and surprisingly capable of greater therapeutic index, and accordingly, have the potential to be superior pharmaceutical agents for the treatment of JAK-mediated disorders.

### SUMMARY OF THE INVENTION

The present invention relates to pyrrolo[2,3-d]pyrimidine compounds and isolated forms of such compounds. The isolated compounds of the invention have been found to possess unexpectedly advantageous activity including higher efficacy and reduced incidence of adverse effects. The isolated compounds may be employed in pharmaceutical compositions in combination with pharmaceutically acceptable excipients and in methods of treating conditions mediated by JAK1.

Disclosed herein are compounds of formula I having the structure: 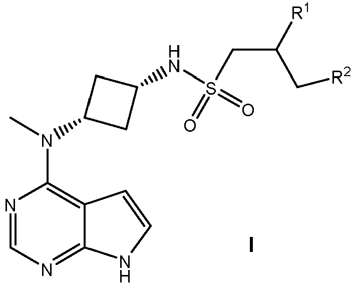 or a pharmaceutically acceptable salt thereof, wherein R¹ and R² are independently hydrogen or hydroxy; wherein if R¹ is hydrogen, then R² is hydroxy; and, wherein if R² is hydrogen, then R¹ is hydroxy.

The invention relates to certain compounds of formula I having the structure of formula IA or formula IC: 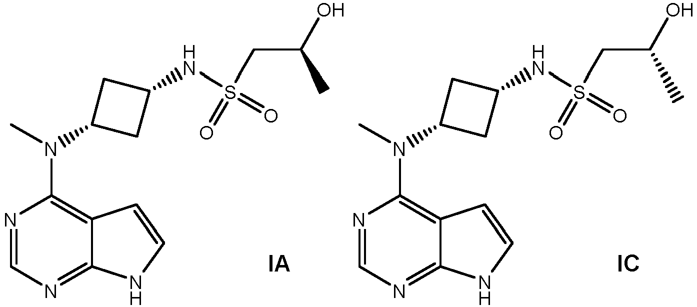 or a pharmaceutically acceptable salt thereof

In other aspects, the present invention also provides compounds of formula IA or IC or a pharmaceutically acceptable salt in an isolated form.

In other aspects, the present invention also provides pharmaceutical compositions which comprise a pharmaceutically acceptable excipient and a compound of formula IA or IC or a pharmaceutically acceptable salt thereof.

In another aspect the present invention also provides a compound of formula IA or IC, or a pharmaceutically acceptable salt thereof, for use in methods for treating conditions or disorders including myositis, vasculitis, pemphigus, Crohn's disease, lupus, nephritis, psoriasis, multiple sclerosis, major depression disorder, allergy, asthma, Sjogren's disease, dry eye syndrome, transplant rejection, cancer, inflammatory bowel disease, septic shock, cardiopulmonary dysfunction, acute respiratory disease, or cachexia, the method comprising administering to a subject in need thereof a therapeutically effective amount of a compound of formula IA or IC or a pharmaceutically acceptable salt.

In another aspect, the present invention also provides a compound of formula IA or IC, or a pharmaceutically acceptable salt thereof, for use in methods for treating conditions or disorders including atopic dermatitis, eczema, psoriasis, scleroderma, lupus, pruritus, other pruritic conditions, allergic reactions including allergic dermatitis in mammal, inflammatory airway disease, recurrent airway obstruction, airway hyper-responsiveness, and chronic obstruction pulmonary disease, the method comprising administering to a subject in need thereof a therapeutically effective amount of a compound of formula IA or IC, or a pharmaceutically acceptable salt thereof.

The term "subject" refers to human, livestock or companion animals.

The term "treating" or "treatment" means an alleviation of symptoms associated with a disease, disorder or condition, or halt of further progression or worsening of those symptoms. Depending on the disease and condition of the subject, the term "treatment" as used herein may include one or more of curative, palliative and prophylactic treatment. Treatment can also include administering a pharmaceutical formulation of the present invention in combination with other therapies.

The term "therapeutically effective" indicates the capability of an agent to prevent, or improve the severity of, the disorder, while avoiding adverse side effects typically associated with alternative therapies. The phrase "therapeutically effective" is to be understood to be equivalent to the phrase "effective for the treatment, prevention, or amelioration", and both are intended to qualify the amount of each agent for use in the combination therapy which will achieve the goal of improvement in the severity of cancer, cardiovascular disease, or pain and inflammation and the frequency of incidence over treatment of each agent by itself, while avoiding adverse side effects typically associated with alternative therapies.

"Pharmaceutically acceptable" means suitable for use in a subject.

If substituents are described as being "independently selected" from a group, each substituent is selected independent of the other. Each substituent therefore may be identical to or different from the other substituent(s). In some embodiments, the metabolite compounds, or salts thereof, are substantially isolated.

The terms "isolated", "purified", "in purified form", "in isolated form" or "purified substance" means that a compound, or salt thereof, for a compound refers to the physical state of the compound after being isolated from a synthetic process, *e.g.,* from a reaction mixture. Thus the terms "isolated", "purified", "in purified form", "in isolated form" or "purified substance" for a compound refers to the physical state of said compound after being obtained from a purification process or processes described herein or well known to the skilled artisan (*e.g.,* chromatography, recrystallization and the like), in sufficient purity to be characterizable by standard analytical techniques described herein or well known to the skilled artisan. As examples, the purification techniques disclosed herein (*e.g.,* LC-MS and LC-MS/MS techniques) result in isolated forms of the subject compounds. Such isolation and purification techniques would be expected to result in product purities containing at least about 70%, at least about 80%, at least about 90%, at least about 95%, at least about 97%, or at least about 99% by weight of the compound, or salt thereof.

### DETAILED DESCRIPTION OF THE INVENTION

The present invention is related to compounds which are selective JAK1 modulators useful for the treatment of diseases and conditions associated with dysregulation of the JAK1. The present invention further provides isolated compounds which are selective JAK1 modulators useful for the treatment of diseases and conditions associated with dysregulation of the JAK1. The present invention also provides pharmaceutical compositions comprising such JAK1 modulators as well as such JAK1 modulators for use in methods of treating and/or preventing such diseases and conditions.

Described below are embodiments (E) of the invention.
E1. A compound of formula IA or formula IC: 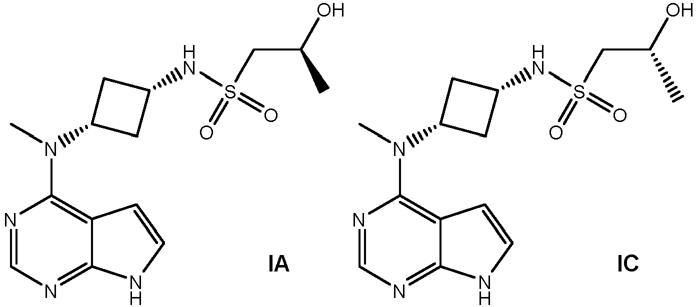 or a pharmaceutically acceptable salt thereof
E4. A compound of formula IA having the structure: 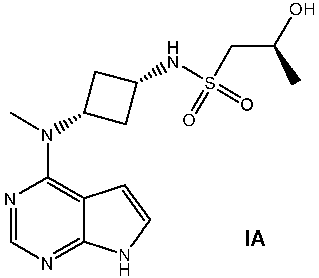 or a pharmaceutically acceptable salt thereof.
E7. (S)-2-Hydroxy-N-(3-(methyl(7H-pyrrolo[2,3-d]pyrimidin-4-yl)amino)cyclobutyl)propane-1-sulfonamide or a pharmaceutically acceptable salt thereof.
E9. (S)-2-Hydroxy-N-(3-(methyl(7H-pyrrolo[2,3-d]pyrimidin-4-yl)amino)cyclobutyl)propane-1-sulfonamide.
E11. The compound according to any one of embodiments E1, E4, E7 or E9, in an isolated form.
E12. The compound according to any one of embodiments E1, E4, E7 or E9, in crystalline form.
E13. A compound according to any one of embodiments E1, E4, E7 or E9 for use in a method or preventing of treating a disease or condition for which a JAK1 inhibitor is indicated, in a subject in need of such treatment, the method comprising administering to the subject a therapeutically effective amount of the compound according to any one of embodiments E1, E4, E7 or E9, or a pharmaceutically acceptable salt.
E14. A compound according to any one of embodiments E1, E4, E7 or E9 for use in a method of treating or preventing an inflammatory or autoimmune condition, the method comprising administering to a subject suffering therefrom a therapeutically effective amount of the compound according to any one of embodiments E1, E4, E7 or E9 or a pharmaceutically acceptable salt thereof.
E15. A pharmaceutical composition comprising a compound according to any one of embodiments E1, E4, E7 or E9 and a pharmaceutically acceptable excipient.
E16. A compound according to any one of embodiments E1, E4, E7 or E9 for use in a method of treating or preventing a disease or condition selected from inflammation, autoimmune disease, neuroinflammation, arthritis, rheumatoid arthritis, spondyloarthropathies, systemic lupus erythematous, lupus nephritis, osteoarthritis, gouty arthritis, pain, fever, pulmonary sarcoidosis, silicosis, cardiovascular disease, atherosclerosis, myocardial infarction, thrombosis, congestive heart failure and cardiac reperfusion injury, cardiomyopathy, stroke, ischemia, reperfusion injury, brain edema, brain trauma, neurodegeneration, liver disease, inflammatory bowel disease, Crohn's disease, ulcerative colitis, nephritis, retinitis, retinopathy, macular degeneration, glaucoma, diabetes (type 1 and type 2), diabetic neuropathy, viral and bacterial infection, myalgia, endotoxic shock, toxic shock syndrome, osteoporosis, multiple sclerosis, endometriosis, menstrual cramps, vaginitis, candidiasis, cancer, fibrosis, obesity, muscular dystrophy, polymyositis, dermatomyositis, autoimmune hepatitis, primary biliary cirrhosis, primary sclerosing cholangitis, vitiligo, Alzheimer's disease, skin flushing, eczema, psoriasis, atopic dermatitis, sunburn, keloid, hypertrophic scar, rheumatic diseases, urticaria, discoid lupus, cutaneous lupus, central nervous system lupus, psoriatic arthritis, asthma, allergic asthma, type I interferonopathies including Aicardi-Goutières syndrome and other mendelian diseases of overexpression of type I interferon, primary progressive multiple sclerosis, relapsing remitting multiple sclerosis, non-alcoholic fatty liver disease, non-alcoholic steatohepatitis, scleroderma, alopecia areata, scarring alopecia, prurigo, prurigo nodularis, CPUO, lichen diseases, lichen planus, Steven's Johnson's syndrome, spondylopathy, myositis, vasculitis, pemphigus, lupus, major depression disorder, allergy, dry eye syndrome, transplant rejection, cancer, septic shock, cardiopulmonary dysfunction, acute respiratory disease, ankylosing spondylitis, cachexia, chronic graft-versus-host disease, acute graft-versus-host disease, Celiac Sprue, idiopathic thrombocytopenic thrombotic purpura, thrombotic thrombocytopenic purpura, myasthenia gravis, Sjogren's syndrome, epidermal hyperplasia, cartilage inflammation, bone degradation, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile Reter's Syndrome, SEA Syndrome, juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, enteropathic arthritis, reactive arthritis, Reter's Syndrome, myolitis, polymyolitis, dermatomyolitis, polyarteritis nodosa, Wegener's granulomatosis, arteritis, polymyalgia rheumatica, sarcoidosis, sclerosis, primary biliary sclerosis, sclerosing cholangitis, dermatitis, Still's disease, chronic obstructive pulmonary disease, Guillain-Barre disease, Graves' disease, Addison's disease, Raynaud's phenomenon, psoriatic epidermal hyperplasia, plaque psoriasis, guttate psoriasis, inverse psoriasis, pustular psoriasis, erythrodermic psoriasis, an immune disorder associated with or arising from activity of pathogenic lymphocytes, noninfectious uveitis, Behcet's disease and Vogt-Koyanagi-Harada syndrome, the method comprising administering to a subject in need thereof a therapeutically effective amount of a compound or a pharmaceutically acceptable salt thereof according to any one of embodiments E1, E4, E7 or E9.
E17. A compound according to any one of embodiments E1, E4, E7 or E9 for use in a method for treating or preventing psoriasis, the method comprising the step of administering to a subject in need thereof a therapeutically effective amount of a composition comprising a compound or a pharmaceutically acceptable salt thereof according to any one of embodiments E1, E4, E7 or E9.
E18. A compound according to any one of embodiments E1, E4, E7 or E9 for use in a method for treating or preventing atopic dermatitis, the method comprising the step of administering to a subject in need thereof a therapeutically effective amount of a composition comprising a compound or a pharmaceutically acceptable salt thereof according to any one of embodiments E1, E4, E7 or E9.
E19. A compound according to any one of embodiments E1, E4, E7 or E9 for use in a method for treating or preventing hand eczema, the method comprising the step of administering to a subject in need thereof a therapeutically effective amount of a compound or a pharmaceutically acceptable salt thereof according to any one of embodiments E1, E4, E7 or E9.
E20. A compound according to any one of embodiments E1, E4, E7 or E9 for use in a method for treating or preventing pruritis, the method comprising the step of administering to a subject in need thereof a therapeutically effective amount of a compound or a pharmaceutically acceptable salt thereof according to any one of embodiments E1, E4, E7 or E9.
E21. A compound according to any one of embodiments E1, E4, E7 or E9 for use in a method for treating or preventing cutaneous lupus, the method comprising the step of administering to a subject in need thereof a therapeutically effective amount of a compound or a pharmaceutically acceptable salt thereof according to any one of embodiments E1, E4, E7 or E9.
E22. A compound according to any one of embodiments E1, E4, E7 or E9 for use in a method of treating a disorder or condition related to dysregulation of JAK, and particularly of JAK1, in a subject in need thereof, the method comprising administering to the subject a therapeutically effective amount of the compound or a pharmaceutically acceptable salt thereof according to any one of embodiments E1, E4, E7 or E9.
E23. The compound for use in a method according to E22, wherein the disorder or condition treated or prevented is selected from the group consisting of inflammation, autoimmune disease, neuroinflammation, arthritis, rheumatoid arthritis, spondyloarthropathies, systemic lupus erythematous, lupus nephritis, osteoarthritis, gouty arthritis, pain, fever, pulmonary sarcoidosis, silicosis, cardiovascular disease, atherosclerosis, myocardial infarction, thrombosis, congestive heart failure and cardiac reperfusion injury, cardiomyopathy, stroke, ischemia, reperfusion injury, brain edema, brain trauma, neurodegeneration, liver disease, inflammatory bowel disease, Crohn's disease, ulcerative colitis, nephritis, retinitis, retinopathy, macular degeneration, glaucoma, diabetes (type 1 and type 2), diabetic neuropathy, viral and bacterial infection, myalgia, endotoxic shock, toxic shock syndrome, osteoporosis, multiple sclerosis, endometriosis, menstrual cramps, vaginitis, candidiasis, cancer, fibrosis, obesity, muscular dystrophy, polymyositis, dermatomyositis, autoimmune hepatitis, primary biliary cirrhosis, primary sclerosing cholangitis, vitiligo, Alzheimer's disease, skin flushing, eczema, psoriasis, atopic dermatitis, sunburn, keloid, hypertrophic scar, rheumatic diseases, urticaria, discoid lupus, cutaneous lupus, central nervous system lupus, psoriatic arthritis, asthma, allergic asthma, type I interferonopathies including Aicardi-Goutières syndrome and other mendelian diseases of overexpression of type I interferon, primary progressive multiple sclerosis, relapsing remitting multiple sclerosis, non-alcoholic fatty liver disease, non-alcoholic steatohepatitis, scleroderma, alopecia areata, spondylopathy, myositis, vasculitis, pemphigus, lupus, major depression disorder, allergy, dry eye syndrome, transplant rejection, cancer, septic shock, cardiopulmonary dysfunction, acute respiratory disease, ankylosing spondylitis, cachexia, chronic graft-versus-host disease, acute graft-versus-host disease, Celiac Sprue, idiopathic thrombocytopenic thrombotic purpura, myasthenia gravis, Sjogren's syndrome, epidermal hyperplasia, cartilage inflammation, bone degradation, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile Reter's Syndrome, SEA Syndrome, juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, enteropathic arthritis, reactive arthritis, Reter's Syndrome, myolitis, polymyolitis, dermatomyolitis, polyarteritis nodosa, Wegener's granulomatosis, arteritis, polymyalgia rheumatica, sarcoidosis, sclerosis, primary biliary sclerosis, sclerosing cholangitis, dermatitis, Still's disease, chronic obstructive pulmonary disease, Guillain-Barre disease, Graves' disease, Addison's disease, Raynaud's phenomenon, psoriatic epidermal hyperplasia, plaque psoriasis, guttate psoriasis, inverse psoriasis, pustular psoriasis, erythrodermic psoriasis, an immune disorder associated with or arising from activity of pathogenic lymphocytes, noninfectious uveitis, Behcet's disease and Vogt-Koyanagi-Harada syndrome.
E24. The compound for use in a method according to embodiments E13 to E23, wherein the therapeutically effective amount is from 0.01 mg/kg of body weight/day to 100 mg/kg of body weight/day.
E25. The compound for use in a method according to E24, wherein the therapeutically effective amount is from 0.1 mg/kg of body weight/day to 10 mg/kg of body weight/day.
E32. A pharmaceutical combination comprising a compound of embodiments E1, E4, E7 or E9, or a pharmaceutically acceptable salt thereof, and one or more additional pharmacologically active compounds.

Compounds or compounds in an isolated form that have the same molecular formula but differ in the nature or sequence of bonding of their atoms or the arrangement of their atoms in space are termed "isomers". Isomers that differ in the arrangement of their atoms in space are termed "stereoisomers". It will be appreciated by those skilled in the art that the compound or compound in an isolated form of formula I, IA or IB can exist as *cis-* and *trans-* achiral diastereomers. An example is the compound or compound in an isolated form of formula IC.

Included within the scope of the described compounds and compounds in an isolated form are all isomers (*e.g., cis-, trans-,* or diastereomers) of the compounds described herein alone as well as any mixtures. All of these forms, including enantiomers, diastereomers, cis, trans, syn, anti, solvates (including hydrates), tautomers, and mixtures thereof, are included in the described compounds or compounds in an isolated form. Stereoisomeric mixtures, *e.g.,* mixtures of diastereomers, can be separated into their corresponding isomers in a known manner by means of suitable separation methods. Diastereomeric mixtures for example may be separated into their individual diastereomers by means of fractionated crystallization, chromatography, solvent distribution, and similar procedures. This separation may take place either at the level of one of the starting compounds or in a compound of formula I, IA or IB itself. Enantiomers may be separated through the formation of diastereomeric salts, for example by salt formation with an enantiomer-pure chiral acid, or by means of chromatography, for example by HPLC, using chromatographic substrates with chiral ligands.

In therapeutic use for treating disorders in a subject, a compound or compound in an isolated form of the present invention or its pharmaceutical compositions can be administered orally, parenterally, topically, rectally, transmucosally, or intestinally. Parenteral administrations include indirect injections to generate a systemic effect or direct injections to the afflicted area. Topical administrations include the treatment of skin or organs readily accessible by local application, for example, eyes or ears. It also includes transdermal delivery to generate a systemic effect. The rectal administration includes the form of suppositories. The preferred routes of administration are oral and parenteral.

Pharmaceutically acceptable salts of the compounds or compounds in an isolated form of formula I, IA or IB include the acid addition and base salts thereof. Suitable acid addition salts are formed from acids which form non-toxic salts. Examples include the acetate, adipate, aspartate, benzoate, besylate, bicarbonate/carbonate, bisulphate/sulfate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts. For a review on suitable salts, see Handbook of Pharmaceutical Salts: Properties, Selection, and Use by Stahl and Wermuth (Wiley-VCH, 2002).

Pharmaceutically acceptable salts of compounds and compounds in an isolated form of formula I, IA or IB may be prepared, respectively, by one or more of three methods: (i) by reacting the compound or compound in an isolated form of formula I, IA or IB with the desired acid or base; (ii) by removing an acid- or base-labile protecting group from a suitable precursor of the compound or compound in an isolated form of formula I, IA or IB or by ring-opening a suitable cyclic precursor, for example, a lactone or lactam, using the desired acid or base; or (iii) by converting one salt of the compound or compound in an isolated form of formula I, IA or IB to another by reaction with an appropriate acid or base or by means of a suitable ion exchange column. All three reactions are typically carried out in solution. The resulting salt may precipitate out and be collected by filtration or may be recovered by evaporation of the solvent. The degree of ionization in the resulting salt may vary from completely ionized to almost non-ionized.

Pharmaceutical compositions of the present invention may be manufactured by methods well known in the art, *e.g.,* by means of conventional mixing, dissolving, granulation, dragee-making, levigating, emulsifying, encapsulating, entrapping, lyophilizing processes or spray drying.

Pharmaceutical compositions for use in accordance with the present invention may be formulated in conventional manner using one or more pharmaceutically acceptable carriers comprising excipients and auxiliaries, which facilitate processing of the active compound or compound in an isolated form into preparations, which can be used pharmaceutically. Proper formulation is dependent upon the route of administration chosen. Pharmaceutically acceptable excipients and carriers are generally known to those skilled in the art and are thus included in the instant invention. Such excipients and carriers are described, for example, in "Remington's Pharmaceutical Sciences" Mack Pub. Co., New Jersey (1991). The formulations of the invention can be designed to be short-acting, fast-releasing, long-acting, and sustained-releasing. Thus, the pharmaceutical formulations can also be formulated for controlled release or for slow release.

Pharmaceutical compositions suitable for use in the present invention include compositions wherein the active ingredients are contained in an amount sufficient to achieve the intended purpose, *i.e.,* control or the treatment of disorders or diseases. More specifically, a therapeutically effective amount means an amount of compound or compound in an isolated form effective to prevent, alleviate or ameliorate symptoms/signs of disease or prolong the survival of the subject being treated.

The quantity of active component, which is the compound or compound in an isolated form of this invention, in the pharmaceutical composition and unit dosage form thereof, may be varied or adjusted widely depending upon the manner of administration, the potency of the particular compound or compound in an isolated form and the desired concentration. Determination of a therapeutically effective amount is well within the capability of those skilled in the art. Generally, the quantity of active component will range between 0.01% to 99% by weight of the composition.

Generally, a therapeutically effective amount of dosage of the active component will be in the range of about 0.01 to about 100 mg/kg of body weight/day, preferably about 0.1 to about 10 mg/kg of body weight/day, more preferably about 0.3 to 3 mg/kg of body weight/day, even more preferably about 0.3 to 1.5 mg/kg of body weight/day It is to be understood that the dosages may vary depending upon the requirements of each subject and the severity of the disorders or diseases being treated.

The desired dose may conveniently be presented in a single dose or as divided doses administered at appropriate intervals, for example, as two, three, four or more sub-doses per day. The sub-dose itself may be further divided, *e.g.,* into a number of discrete loosely spaced administrations; such as multiple inhalations from an insufflator or by application of a plurality of drops into the eye.

Also, it is to be understood that the initial dosage administered may be increased beyond the above upper level in order to rapidly achieve the desired plasma concentration. On the other hand, the initial dosage may be smaller than the optimum and the daily dosage may be progressively increased during the course of treatment depending on the particular situation. If desired, the daily dose may also be divided into multiple doses for administration, *e.g.,* two to four times per day.

Compounds and compounds in an isolated form of the present invention may be administered in a pharmaceutically acceptable form either alone or in combination with one or more additional agents which modulate a mammalian immune system or with anti-inflammatory agents. These agents may include but are not limited to a 5-lipoxygenase activating protein (FLAP) antagonist; a leukotriene antagonist (LTRA) such as an antagonist of LTB4, LTC4, LTD4, LTE4, CysLT1 or CysLT2, *e.g.,* montelukast or zafirlukast; a histamine receptor antagonist, such as a histamine type 1 receptor antagonist or a histamine type 2 receptor antagonist, *e.g.,* loratidine, fexofenadine, desloratidine, levocetirizine, methapyrilene or cetirizine; an α1-adrenoceptor agonist or an α2-adrenoceptor agonist, e.g., phenylephrine, methoxamine, oxymetazoline or methylnorephrine; a muscarinic M3 receptor antagonist, e.g. tiotropium or ipratropium; a dual muscarinic M3 receptor antagononist/β2 agonist; a PDE inhibitor, such as a PDE3 inhibitor, a PDE4 inhibitor or a PDE5 inhibitor, *e.g.,* theophylline, sildenafil, vardenafil, tadalafil, ibudilast, cilomilast or roflumilast; sodium cromoglycate or sodium nedocromil; a cyclooxygenase (COX) inhibitor, such as a non-selective inhibitor (*e.g.,* aspirin or ibuprofen) or a selective inhibitor (e.g. celecoxib or valdecoxib); a glucocorticosteroid, *e.g.,* fluticasone, mometasone, dexamethasone, prednisolone, budesonide, ciclesonide or beclamethasone; an anti-inflammatory monoclonal antibody, *e.g.,* infliximab, adalimumab, tanezumab, ranibizumab, bevacizumab or mepolizumab; a β2 agonist, *e.g.,* salmeterol, albuterol, salbutamol, fenoterol or formoterol, particularly a long-acting β2 agonist; an integrin antagonist, *e.g.,* natalizumab; an adhesion molecule inhibitor, such as a VLA-4 antagonist; a kinin B1 or B2 receptor antagonist; an immunosuppressive agent, such as an inhibitor of the IgE pathway (*e.g.,* omalizumab) or cyclosporine; a matrix metalloprotease (MMP) inhibitor, such as an inhibitor of MMP-9 or MMP-12; a tachykinin NK1, NK2 or NK3 receptor antagonist; a protease inhibitor, such as an inhibitor of elastase, chymase or catheopsin G; an adenosine A2a receptor agonist; an adenosine A2b receptor antagonist; a urokinase inhibitor; a dopamine receptor agonist (*e.g.,* ropinirole), particularly a dopamine D2 receptor agonist (*e.g.,* bromocriptine); a modulator of the NFKB pathway, such as an IKK inhibitor; a further modulator of a cytokine signaling pathway such as an inhibitor of JAK kinase, syk kinase, p38 kinase, SPHK-1 kinase, Rho kinase, EGF-R or MK-2; a mucolytic, mucokinetic or anti-tussive agent; an antibiotic; an antiviral agent; a vaccine; a chemokine; an epithelial sodium channel (ENaC) blocker or Epithelial sodium channel (ENaC) inhibitor; a nucleotide receptor agonist, such as a P2Y2 agonist; a thromboxane inhibitor; niacin; a 5-lipoxygenase (5-LO) inhibitor, e.g., Zileuton; an adhesion factor, such as VLAM, ICAM or ELAM; a CRTH2 receptor (DP2) antagonist; a prostaglandin D2 receptor (DP1) antagonist; a haematopoietic prostaglandin D2 synthase (HPGDS) inhibitor; interferon-β; a soluble human TNF receptor, *e.g.,* Etanercept; a HDAC inhibitor; a phosphoinositotide 3-kinase gamma (Pl3Kγ) inhibitor; a phosphoinositide 3-kinase delta (Pl3Kδ) inhibitor; a CXCR-1 or a CXCR-2 receptor antagonist; an IRAK-4 inhibitor; and, a TLR-4 or TLR-9 inhibitor, including the pharmaceutically acceptable salts of the specifically named compounds. The agents may be administered with another active agent, wherein the second active agent may be administered either orally or topically.

Suitable specific agents for use in combination therapy with a compound or compound in an isolated form of formula I, IA or IB, or a pharmaceutically acceptable salt thereof, include sulfasalazine, mesalazine, prednisone, azathioprine, infliximab, adalimumab, belimumab, becertolizumab, natalizumab, vedolizumab, hydrocortisone, budesonide, cyclosporin, tacrolimus, fexofenadine, 6-mercaptopurine, methotrexate, ursodeoxycholic acid, obeticholic acid, antihistamines, rifampin, prednisone , methotrexate, azathioprine, cyclophosphamide, hydroxychloroquine, mofetil, sodium mycophenolate, tacrolimus, leflunomide, chloroquine and quinacrine, thalidomide, rituxan, NSAIDs, solumedrol, depomedrol and dexamethasone.

### Chemical Synthesis

The following schemes and written descriptions provide general details regarding the preparation of the compounds of the invention and reference compounds.

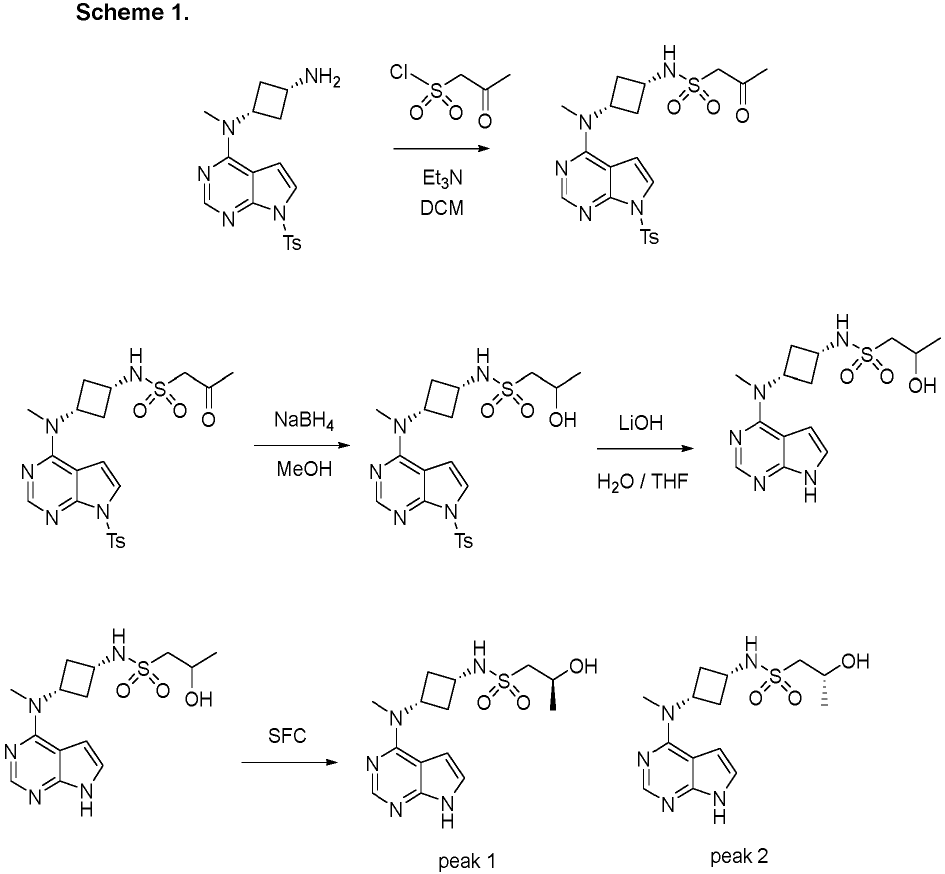

A synthetic route relating to both Example 1 and Example 2 is depicted in Scheme 1. The primary amine (J. Med. Chem. 2018, 61(3), 1130-1152) is treated with 2-oxo-1-propanesulfonyl chloride (prepared in two steps from chloroacetone) in dichloromethane containing triethylamine. The resulting sulfonamide is then treated with sodium borohydride in methanol to reduce the ketone and afford an isomeric mixture of secondary alcohols. The tosyl protecting is then removed from the pyrrolopyrimidine ring. The secondary alcohols are then separated via SFC to afford the compound formed in Example 1 (peak 1) corresponding to compound IA and and the compound formed in Example 2 (peak 2), under the conditions described.

An alternative preparation of Example 1 is depicted in Scheme 2.

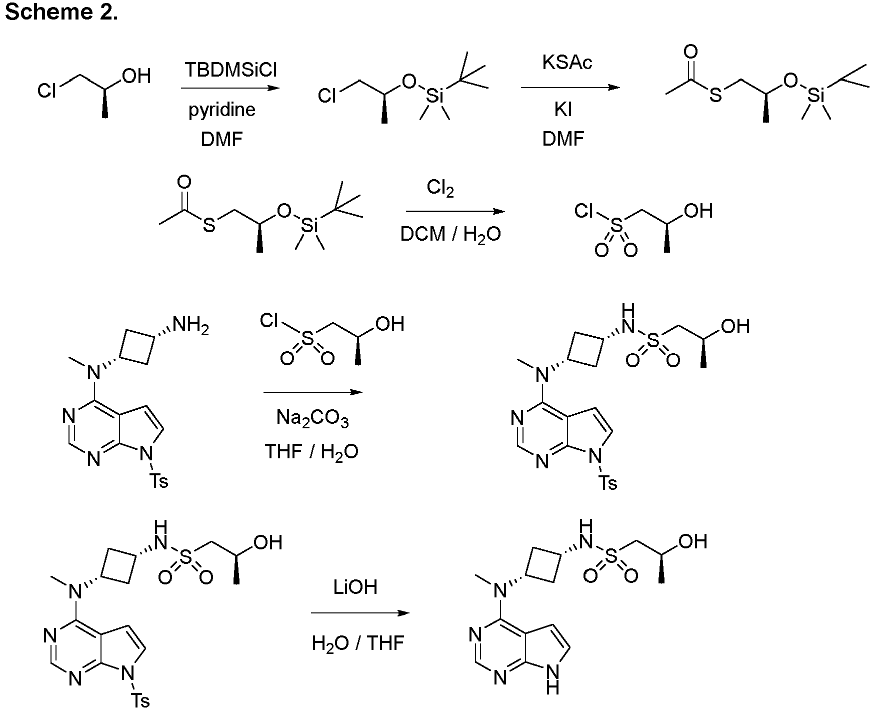

(*S*)-1-Chloro-2-propanol is protected as a silyl ether using *tert*-butyldimethylsilyl chloride in dimethyl formamide as the solvent and pyridine as the base. The primary chloride is displaced with potassium thioacetate to give the thioester. The thioester is oxidized with chlorine gas to give the sulfonyl chloride. Under the reaction conditions, the silyl protecting group can be concomitantly removed. *cis*-N-Methyl-N-{7-[(4-methylphenyl)sulfonyl]-7H-pyrrolo[2,3-d]pyrimidin-4-yl}cyclobutane-1,3-diamine is reacted with the sulfonyl chloride to afford the sulfonamide. The tosyl protecting group is then removed from the pyrrolopyrimidine using lithium hydroxide in a mixture of water and tetrahydrofuran to yield compound **IA** (Example 1) after SFC purification.

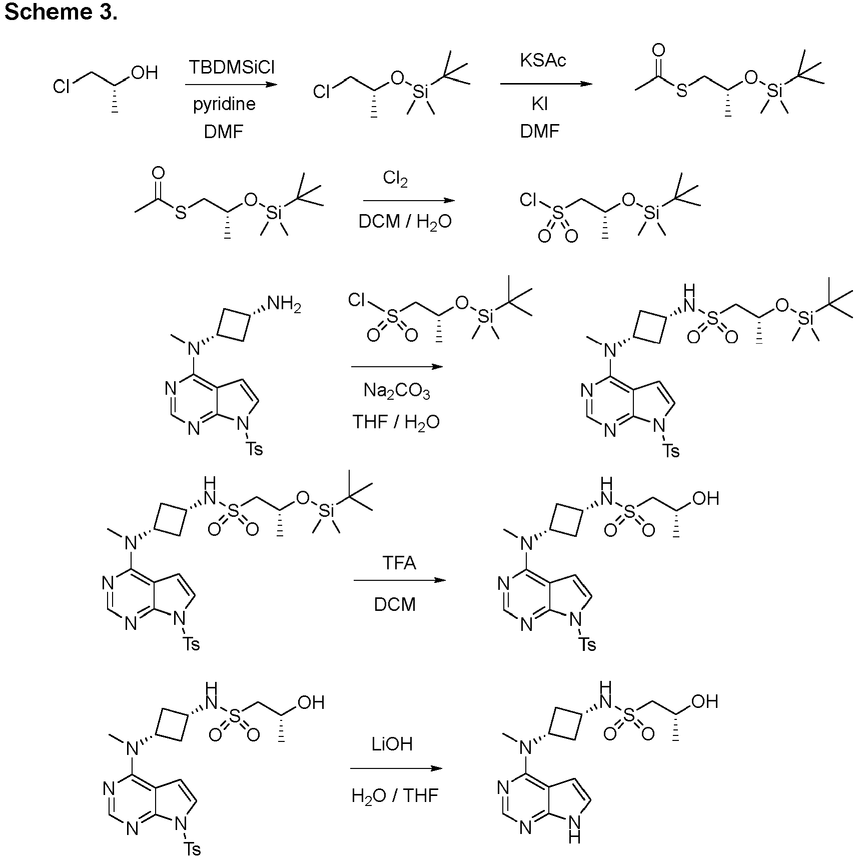

(*R*)-1-Chloro-2-propanol is protected as a silyl ether using *tert*-butyldimethylsilyl chloride in dimethyl formamide as the solvent and pyridine as the base. The primary chloride is displaced with potassium thioacetate to give the thioester. The thioester is oxidized with chlorine gas to give the sulfonyl chloride; some loss of the silyl protecting group is observed. *cis*-N-Methyl-N-{7-[(4-methylphenyl)sulfonyl]-7H-pyrrolo[2,3-d]pyrimidin-4-yl}cyclobutane-1,3-diamine is reacted with the sulfonyl chloride to afford the sulfonamide. The silyl protecting group is cleaved with trifluoroacetic acid to give the secondary alcohol. The tosyl protecting group is then removed from the pyrrolopyrimidine using lithium hydroxide in a mixture of water and tetrahydrofuran to yield the compound of Example 2 after SFC purification.

A preparation of the reference compound IB is depicted in Scheme 4, as set forth in Example 5. *cis*-N-Methyl-N-{7-[(4-methylphenyl)sulfonyl]-7H-pyrrolo[2,3-d]pyrimidin-4-yl}cyclobutane-1,3-diamine is reacted with methyl 3-(chlorosulfonyl)propanoate to afford the sulfonamide. The sulfonamide was reduced with lithium aluminum hydride to afford 3-hydroxy-N-(cis-3-(methyl(7H-pyrrolo[2,3-d]pyrimidin-4-yl)amino)cyclobutyl)propane-1-sulfonamide (IB).

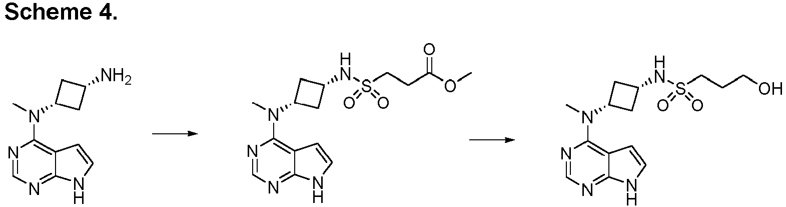

An alternative preparation of the reference compound IB is depicted in Scheme 5, as set forth in Example 6. cis-N-Methyl-N-{7-[(4-methylphenyl)sulfonyl]-7H-pyrrolo[2,3-d]-pyrimidin-4-yl}cyclobutane-1,3-diamine dihydrobromide was treated with a base such as triethylamine and ethyl 3-(chlorosulfonyl)propanoate to afford ethyl 3-(N-(cis-3-(methyl(7-tosyl-7H-pyrrolo[2,3-d]pyrimidin-4-yl)amino)cyclobutyl)sulfamoyl)propanoate. The latter sulfamoylpropanoate was reduced with lithium aluminum hydride, and worked up to afford the crude product 3-hydroxy-N-(cis-3-(methyl(7-tosyl-7H-pyrrolo[2,3-d]pyrimidin-4-yl)amino)cyclobutyl)propane-1-sulfonamide which was used directly without further purification. The latter sulfonamide was treated with base such as LiOH to give 3-hydroxy-N-(cis-3-(methyl(7H-pyrrolo[2,3-d]pyrimidin-4-yl)amino)cyclobutyl)propane-1-sulfonamide (**IB**).

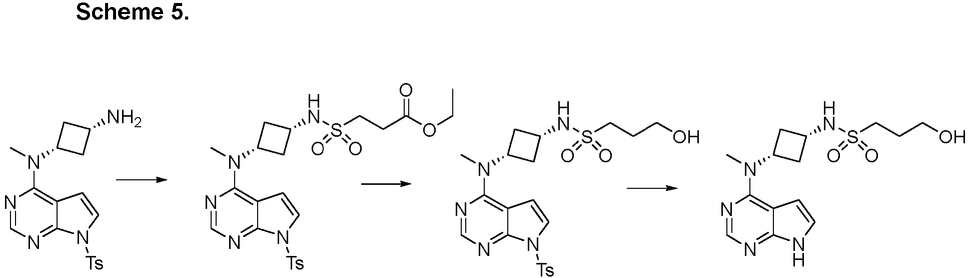

In executing the synthesis of the compounds of the invention, one skilled in the art will recognize the need to sample and assay reaction mixtures prior to work up in order to monitor the progress of reactions and decide whether the reaction should be continued or whether it is ready to be worked up to obtain the desired product. Common methods for assaying reaction mixtures include thin-layer chromatography (TLC), liquid chromatography/mass spectroscopy (LCMS), and nuclear magnetic resonance (NMR).

One skilled in the art will also recognize that the compounds and compounds in an isolated form of the invention may be prepared as mixtures of diastereomers or geometric isomers (*e.g.*, cis and trans substitution on a cycloalkane ring). These isomers can be separated by standard chromatographic techniques, such as normal phase chromatography on silica gel, reverse phase preparative high pressure liquid chromatography or supercritical fluid chromatography. One skilled in the art will also recognize that some compounds of the invention are chiral and thus may be prepared as racemic or scalemic mixtures of enantiomers. Several methods are available and are well known to those skilled in the art for the separation of enantiomers. A preferred method for the routine separation enantiomers is supercritical fluid chromatography employing a chiral stationary phase.

### EXPERIMENTAL SECTION

Except where otherwise noted, reactions were run under an atmosphere of nitrogen. Chromatography on silica gel was carried out using 250-400 mesh silica gel using pressurized nitrogen (~10-15 psi) to drive solvent through the column ("flash chromatography"). Where indicated, solutions and reaction mixtures were concentrated by rotary evaporation under vacuum.

### Preparations and Examples.

### Preparation 1. 2-oxopropane-1-sulfonic acid, sodium salt

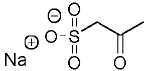

A mixture of chloroacetone (9.25 g, 100 mmol) and sodium sulphite (14.5 g, 115 mmol) in water (100 mL) was stirred for 24 h. The mixture was concentrated to dryness and the resulting solids suspended in methanol (300 mL). The mixture was sonicated for 3 minutes then filtered. The cake was washed with methanol and the filtrates concentrated to give the title compound (15.5 g) which was used directly without further purification.

### Preparation 2. 2-oxopropane-1-sulfonyl chloride

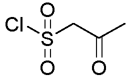

To a stirred suspension of 2-oxopropane-1-sulfonic acid sodium salt (15.5 g, 97 mmol) in dry toluene (40 mL) was added phosphorus oxychloride (40 mL). The mixture was refluxed for 3 h then removed from heat and chilled to 20°C. The mixture was concentrated under reduced pressure and the residue treated with dichloromethane (100 mL). The mixture was filtered and the cake washed with dichloromethane (20 mL). The filtrate was concentrated to give the title compound (11.8 g). ¹H NMR (400 MHz, CDCl₃-d) δ 4.76 - 4.53 (m, 2H), 2.49 (s, 3H).

### Preparation 3. cis-N-(3-(Methyl(7-tosyl-7H-pyrrolo[2,3-d]pyrimidin-4-yl)amino)cyclobutyl)-2-oxopropane-1-sulfonamide

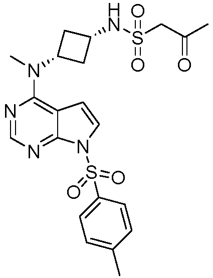

To a solution of *cis-*N¹-methyl-N¹-(7-tosyl-7H-pyrrolo[2,3-d]pyrimidin-4-yl)cyclobutane-1,3-diamine (2.4 g, 5.3 mmol) in dichloromethane (45 mL) was added triethylamine (2.68 g, 26.5 mmol). A solution of 2-oxopropane-1-sulfonyl chloride (1.24 g, 6.27 mmol) in dichloromethane (5 mL) was added at 0°C and the mixture was allowed to warm to 20°C and stirred for 3 h. The mixture was quenched with saturated ammonium chloride (30 mL). The layers were separated, and the aqueous layer was extracted twice with dichloromethane (2 x 10 mL). The combined organic layers were washed with brine, dried over sodium sulfate and concentrated under reduced pressure. The residue was purified via flash chromatography using (petroleum ether:ethyl acetate, 1:2) to give the title compound (801 mg). LC/MS [M+H] = 491.9.

### Preparation 4. 2-Hydroxy-N-(cis-3-(methyl(7-tosyl-7H-pyrrolo[2,3-d]pyrimidin-4-yl)amino)cyclobutyl)propane-1-sulfonamide

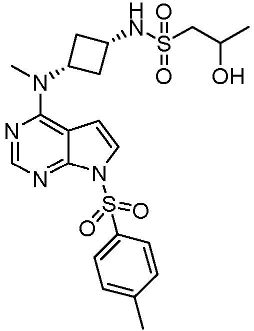

To a solution of N-(3-{methyl[7-(4-methylbenzene-1-sulfonyl)-7H-pyrrolo[2,3-d]pyrimidin-4-yl]amino}cyclobutyl)-2-oxopropane-1-sulfonamide (0.801 g, 1.62 mmol) in methanol (10 mL) was added sodium borohydride (123 mg, 2.26 mmol) in 10% sodium hydroxide (1.6 mL) and methanol (1.6 mL) at 0°C. The mixture was stirred for 30 minutes and then removed from the cooling bath and stirred at 20°C for 30 minutes. The methanol was removed under reduced pressure. The residue was dissolved in ethyl acetate (15 mL), washed with brine (10 mL), dried over sodium sulfate and concentrated under reduced pressure. The residue was purified via flash chromatography using 0-80% ethyl acetate in petroleum ether to give the title compound (0.76 g). LC/MS [M+H] = 494.2.

### Preparation 5. 2-Hydroxy-N-(cis-3-(methyl(7H-pyrrolo[2,3-d]pyrimidin-4-yl)amino)cyclobutyl)propane-1-sulfonamide

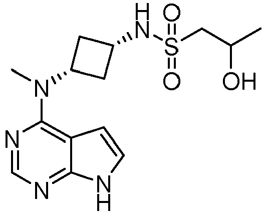

To a mixture of 2-hydroxy-N-(*cis*-3-{methyl[7-(4-methylbenzene-1-sulfonyl)-7H-pyrrolo[2,3-d]pyrimidin-4-yl]amino}cyclobutyl)propane-1-sulfonamide (1.13 g, 2.3 mmol) in tetrahydrofuran (10 mL) and water (10 mL) was added lithium hydroxide monohydrate (273 mg, 11.4 mmol) at 20°C for 24 h. The mixture was concentrated and purified via flash chromatography using 0-10% methanol in dichloromethane. The mixture was then dissolved in water/acetonitrile then lyophilized. The residue was purified via prep thin layer chromatography (dichloromethane:methanol, 10:1). The residue was dissolved in water/acetonitrile and lyophilized. The residue was then purified via supercritical fluid chromatography (Chiralpak AD-H^{™} 250x30mm I.D., 5µ Mobile phase: 45% methanol (0.1%) NH₄OH) in CO₂, flow rate 50 mL/min, Temp 35°C, RT 3.53 and 4.07 min.) to give the title compound (552 mg, 71%). ¹H NMR (400 MHz, DMSO-d₆) δ 11.64 (br s, 1H), 8.11 (s, 1H), 7.44 (d, *J* = 9.0 Hz, 1H), 7.16 - 7.15 (m, 1H), 6.65 - 6.64 (m, 1H), 5.02 - 4.81 (m, 2H), 4.15 - 3.94 (m, 1H), 3.67 - 3.54 (m, 1H), 3.26 (s, 3H), 3.14 - 2.95 (m, 2H), 2.71 - 2.56 (m, 2H), 2.31 - 2.19 (m, 2H), 1.22 (d, *J* = 6*.*0 Hz, 3H), LC/MS [M+H] = 339.9.

### Example 1. (S)-2-Hydroxy-N-(cis-3-(methyl(7H-pyrrolo[2,3-d]pyrimidin-4-yl)amino)cyclobutyl)propane-1-sulfonamide (IA)

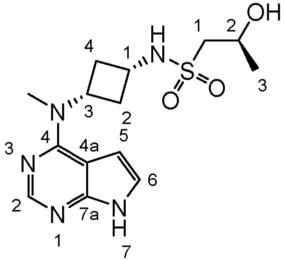

2-hydroxy-N-{3-[methyl(7H-pyrrolo[2,3-d]pyrimidin-4-yl)amino]cyclobutyl}propane-1-sulfonamide (532 mg, 1.57 mmol) was purified via SFC ((Chiralpak AD-H^{™} 250x30mm I.D., 5µ Mobile phase: 45% ethanol (0.1%) NH₄OH) in CO₂, flow rate 50 mL/min, Temp 35°C, RT 3.44min.) to give the title compound (223.7 mg, 42%).¹H NMR (400 MHz, DMSO-d₆) δ = 11.69 - 11.57 (m, 1H), 8.10 (s, 1H), 7.43 (d, *J* = 9.0 Hz, 1H), 7.16 - 7.12 (m, 1H), 6.67 - 6.61 (m, 1H), 4.92 (d, *J =* 5.0 Hz, 1H), 4.90 - 4.84 (m, 1H), 4.11 - 4.01 (m, 1H), 3.63 - 3.53 (m, 1H), 3.27 - 3.23 (m, 3H), 3.11 - 2.96 (m, 2H), 2.63 - 2.53 (m, 2H), 2.29 - 2.20 (m, 2H), 1.21 (d, *J* = 6.0 Hz, 3H), LC/MS [M+H] = 340.2. Crystalline material can be prepared from ethyl acetate.

### Single Crystal X-Ray Structure

Data collection was performed on a Bruker D8 Quest diffractometer at room temperature. Data collection consisted of omega and phi scans. The structure was solved by intrinsic phasing using SHELX software suite in the Triclinic space group P1. The structure was subsequently refined by the full-matrix least squares method. All non-hydrogen atoms were found and refined using anisotropic displacement parameters.

The hydrogen atoms located on nitrogen and oxygen were found from the Fourier difference map and refined with distances restrained. The remaining hydrogen atoms were placed in calculated positions and were allowed to ride on their carrier atoms. The final refinement included isotropic displacement parameters for all hydrogen atoms. Analysis of the absolute structure using likelihood methods (Hooft 2008) was performed using PLATON (Spek 2010). Assuming the sample submitted is enantiopure, the results indicate that the absolute structure has been correctly assigned. The method calculates that the probability that the structure is correctly assigned is 100%. The Hooft parameter is reported as 0.104 with an Esd of (15) and the Parson's parameter is reported as 0.097 with an Esd of (16). Absolute configuration at C13_C27 for two identical molecules per Asymmetric Unit was confirmed as (-S)_(-S). The final R-index was 5.9%. A final difference Fourier revealed no missing or misplaced electron density. Pertinent crystal, data collection and refinement are summarized in Table 1. Atomic coordinates are listed in Table 2.

### Software and References

SHELXTL, Version 5.1, Bruker AXS, 1997. PLATON, A.L. Spek, J. Appl. Cryst. 2003, 36, 7-13. MERCURY, C.F. Macrae, P.R. Edington, P. McCabe, E. Pidcock, G.P. Shields, R. Taylor, M. Towler and J. van de Streek, J. Appl. Cryst. 39, 453-457, 2006. OLEX2, Dolomanov, O.V.; Bourhis, L.J.; Gildea, R.J.; Howard, J.A.K.; Puschmann, H., (2009). J. Appl. Cryst., 42, 339-341. R.W.W. Hooft et al. J. Appl. Cryst. (2008). 41. 96-103. H.D. Flack, Acta Cryst. 1983, A39, 867-881.

**Table 1. Crystal data and structure refinement for IA**

| | | |
|---|---|---|
| Crystallization Solvent: | | Ethyl Acetate - Acetone |
| Empirical formula | | C14 H21 N5 O3 S |
| Formula weight | | 339.42 |
| Temperature | | 296(2) K |
| Wavelength | 1.54178 Å | |
| Crystal system | Triclinic | |
| Space group | P1 | |
| Unit cell dimensions | a = 5.5384(2) Å | α= 88.745(2)°. |
| | b = 6.9183(2) Å | β= 83.850(2)°. |
| | c = 21.8043(7) Å | γ= 79.656(2)°. |
| Volume | 817.15(5) Å³ | |
| Z | 2 | |
| Density (calculated) | 1.379 Mg/m³ | |
| Absorption coefficient | 1.963 mm⁻¹ | |
| F(000) | 360 | |
| Crystal size | 0.140 x 0.120 x 0.020 mm³ | |
| Theta range for data collection | 4.078 to 72.163°. | |
| Index ranges | -6<=h<=5, -8<=k<=8, -26<=I<=26 | |
| Reflections collected | 11795 | |
| Independent reflections | 4989 [R(int) = 0.0466] | |
| Completeness to theta = 67.679° | 98.4 % | |
| Absorption correction | Empirical | |
| Refinement method | Full-matrix least-squares on F² | |
| Data / restraints / parameters | / 9 / 437 | |
| Goodness-of-fit on F² | 1.100 | |
| Final R indices [I>2sigma(I)] | R1 = 0.0589, wR2 = 0.1557 | |
| R indices (all data) | R1 = 0.0610, wR2 = 0.1579 | |
| Absolute structure parameter | 0.091(15) | |
| Extinction coefficient | n/a | |
| Largest diff. peak and hole | 0.339 and -0.483 e.Å⁻³ | |

**Table 2. Atomic coordinates (x 10⁴) and equivalent isotropic displacement parameters (Å²x 10³) for IA. U(eq) is defined as one third of the trace of the orthogonalized U^{ij} tensor.**

| | x | y | z | U(eq) |
|---|---|---|---|---|
| S(1) | 10411(2) | 184(1) | 1306(1) | 44(1) |
| S(2) | -172(2) | 9984(1) | 8832(1) | 50(1) |
| N(1) | 2278(7) | 3071(5) | 4042(2) | 46(1) |
| N(2) | -1535(7) | 3927(5) | 4678(2) | 46(1) |
| N(3) | -3541(8) | 7256(6) | 4570(2) | 49(1) |
| N(4) | 3905(8) | 5099(6) | 3318(2) | 49(1) |
| N(5) | 9233(9) | 2050(6) | 1714(2) | 54(1) |
| N(6) | 8576(7) | 7489(5) | 6091(2) | 46(1) |
| N(7) | 12380(7) | 6612(5) | 5446(2) | 46(1) |
| N(8) | 14370(8) | 3292(6) | 5560(2) | 50(1) |
| N(9) | 6925(8) | 5461(6) | 6813(2) | 49(1) |
| N(10) | 1745(10) | 8319(6) | 8458(2) | 59(1) |
| O(1) | 11777(8) | 841(6) | 779(2) | 63(1) |
| O(2) | 11703(8) | -1282(6) | 1692(2) | 62(1) |
| O(3) | 9634(14) | -4205(6) | 1264(3) | 109(2) |
| O(4) | -1217(8) | 11398(6) | 8398(2) | 59(1) |
| O(5) | -1797(10) | 9021(7) | 9231(2) | 80(1) |
| O(6) | 1326(14) | 14200(7) | 8702(3) | 105(2) |
| C(1) | 500(9) | 2733(7) | 4470(2) | 48(1) |
| C(2) | -1643(8) | 5753(6) | 4425(2) | 42(1) |
| C(3) | 91(8) | 6360(6) | 3992(2) | 40(1) |
| C(4) | -839(11) | 8403(7) | 3882(3) | 56(1) |
| C(5) | -2995(12) | 8885(8) | 4244(3) | 62(1) |
| C(6) | 2119(8) | 4884(6) | 3778(2) | 40(1) |
| C(7) | 3866(13) | 6954(10) | 2995(3) | 80(2) |
| C(8) | 5543(10) | 3357(8) | 3056(2) | 55(1) |
| C(9) | 5176(11) | 2533(10) | 2439(3) | 74(2) |
| C(10) | 7956(10) | 1932(8) | 2329(2) | 54(1) |
| C(11) | 8219(10) | 3427(11) | 2796(3) | 72(2) |
| C(12) | 7928(10) | -831(7) | 1061(3) | 54(1) |
| C(13) | 8819(12) | -2883(7) | 797(3) | 63(1) |
| C(14) | 6800(19) | -3640(12) | 526(4) | 95(3) |
| C(15) | 10333(9) | 7808(6) | 5665(2) | 47(1) |
| C(16) | 12468(8) | 4801(6) | 5706(2) | 39(1) |
| C(17) | 10720(9) | 4202(6) | 6146(2) | 41(1) |
| C(18) | 11687(11) | 2153(7) | 6259(3) | 58(1) |
| C(19) | 13841(12) | 1671(7) | 5903(3) | 63(1) |
| C(20) | 8716(8) | 5678(6) | 6352(2) | 40(1) |
| C(21) | 6983(15) | 3596(9) | 7127(3) | 80(2) |
| C(22) | 5366(10) | 7195(8) | 7091(2) | 55(1) |
| C(23) | 2676(10) | 7212(9) | 7330(2) | 57(1) |
| C(24) | 2987(10) | 8554(8) | 7848(2) | 54(1) |
| C(25) | 5758(11) | 7857(11) | 7732(3) | 77(2) |
| C(26) | 1395(12) | 11250(8) | 9315(2) | 61(1) |
| C(27) | 2857(11) | 12692(8) | 8997(3) | 63(1) |
| C(28) | 4098(16) | 13634(13) | 9462(4) | 91(2) |

### Example 2. (R)-2-Hydroxy-N-(cis-3-(methyl(7H-pyrrolo[2,3-d]pyrimidin-4-yl)amino)cyclobutyl)propane-1-sulfonamide (IC)

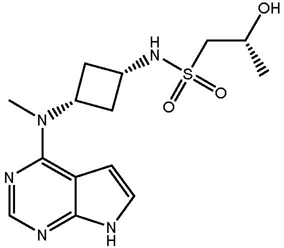

2-hydroxy-N-{3-[methyl(7H-pyrrolo[2,3-d]pyrimidin-4-yl)amino]cyclobutyl}propane-1-sulfonamide (532 mg, 1.57 mmol) was purified via SFC ((Chiralpak AD-H^{™} 250x30mm I.D., 5µ Mobile phase: 45% ethanol (0.1%) NH₄OH) in CO₂, flow rate 50 mL/min, Temp 35°C, RT 3.88min.) to give the title compound (222.7 mg, 42%). ¹H NMR (400 MHz, DMSO-d₆) δ = 11.69 - 11.56 (m, 1H), 8.10 (s, 1H), 7.43 (d, *J* = 9.0 Hz, 1H), 7.18 - 7.11 (m, 1H), 6.66 - 6.60 (m, 1H), 4.96 - 4.84 (m, 2H), 4.12 - 3.99 (m, 1H), 3.66 - 3.54 (m, 1H), 3.24 (s, 3H), 3.12 - 2.94 (m, 2H), 2.64 - 2.56 (m, 2H), 2.30 - 2.18 (m, 2H), 1.20 (d, *J* = 6.5 Hz, 3H), LC/MS [M+H] = 340.2. Crystalline material can be prepared from ethyl acetate.

### Single Crystal X-Ray Structure

Data collection was performed on a Bruker D8 Quest diffractometer at room temperature. Data collection consisted of omega and phi scans. This lot gave plate like crystals stacked together, a crystal was separated and mounted for collection. The structure was solved by intrinsic phasing using SHELX software suite in the Triclinic class space group P1. The structure was subsequently refined by the full-matrix least squares method. All non-hydrogen atoms were found and refined using anisotropic displacement parameters. The hydrogen atoms located on nitrogen and oxygen were found from the Fourier difference map and refined with distances restrained. The remaining hydrogen atoms were placed in calculated positions and were allowed to ride on their carrier atoms. The final refinement included isotropic displacement parameters for all hydrogen atoms. Analysis of the absolute structure using likelihood methods (Hooft 2008) was performed using PLATON (Spek 2010). Assuming the sample submitted is enantiopure, the results indicate that the absolute structure has been correctly assigned. The method calculates that the probability that the structure is correctly assigned is 100.0. The Hooft parameter is reported as 0.044 with an Esd of (18) and the Parson's parameter is reported as 0.050 with an Esd of (19). Absolute configuration at C13 and C27 for two identical molecules per asymmetric Unit was confirmed as (-R)_(-R). Pseudo-symmetry to P-1. The final R-index was 4.5%. A final difference Fourier revealed no missing or misplaced electron density. Pertinent crystal, data collection and refinement are summarized in Table 3. Atomic coordinates are listed in Table 4.

### Software and References

SHELXTL, Version 5.1, Bruker AXS, 1997. PLATON, A.L. Spek, J. Appl. Cryst. 2003, 36, 7-13. MERCURY, C.F. Macrae, P.R. Edington, P. McCabe, E. Pidcock, G.P. Shields, R. Taylor, M. Towler and J. van de Streek, J. Appl. Cryst. 39, 453-457, 2006. OLEX2, Dolomanov, O.V.; Bourhis, L.J.; Gildea, R.J.; Howard, J.A.K.; Puschmann, H., (2009). J. Appl. Cryst., 42, 339-341.R.W.W. Hooft et al. J. Appl. Cryst. (2008). 41. 96-103. H.D. Flack, Acta Cryst. 1983, A39, 867-881.

**Table 3. Crystal data and structure refinement for IC.**

| | | |
|---|---|---|
| Crystallization Solvent | Ethyl Acetate / Acetone | |
| Empirical formula | C14 H21 N5 O3 S | |
| Formula weight | 339.42 | |
| Temperature | 296(2) K | |
| Wavelength | 1.54178 Å | |
| Crystal system | Triclinic | |
| Space group | P1 | |
| Unit cell dimensions | a = 5.5384(2) Å | α= 88.745(2)°. |
| | b = 6.9183(2) Å | β= 83.850(2)°. |
| | c = 21.8043(7) Å | γ = 79.656(2)°. |
| Volume | 817.15(5) Å³ | |
| Z | 2 | |
| Density (calculated) | 1.379 Mg/m³ | |
| Absorption coefficient | 1.963 mm⁻¹ | |
| F(000) | | 360 |
| Crystal size | | 0.140 x 0.120 x 0.020 mm3 |
| Theta range for data collection | | 4.078 to 80.472°. |
| Index ranges | | -5<=h<=6, -8<=k<=8, -27<=I<=27 |
| Reflections collected | | 11873 |
| Independent reflections | | 5335 [R(int) = 0.0440] |
| Completeness to theta = 67.679° | | 96.5 % |
| Absorption correction | | Empirical |
| Refinement method | | Full-matrix least-squares on F² |
| Data / restraints / parameters | | 5335 / 9 / 437 |
| Goodness-of-fit on F² | | 1.019 |
| Final R indices [I>2sigma(I)] | | R1 = 0.0450, wR2 = 0.1085 |
| R indices (all data) | | R1 = 0.0553, wR2 = 0.1161 |
| Absolute structure parameter | | 0.052(18) |
| Extinction coefficient | | n/a |
| Largest diff. peak and hole | | 0.331 and -0.263 e.Å-3 |

**Table 4. Atomic coordinates (x 10⁴) and equivalent isotropic displacement parameters (Å²x 10³) for IB. U(eq) is defined as one third of the trace of the orthogonalized U^{ij} tensor.**

| | x | y | z | U(eq) |
|---|---|---|---|---|
| S(1) | -424(2) | 9823(1) | 8694(1) | 46(1) |
| S(2) | 10185(2) | 11(1) | 1169(1) | 51(1) |
| N(1) | 7725(7) | 6925(5) | 5959(2) | 47(1) |
| N(2) | 11519(7) | 6066(5) | 5323(2) | 48(1) |
| N(3) | 13535(7) | 2746(6) | 5433(2) | 51(1) |
| N(4) | 6084(8) | 4900(6) | 6681(2) | 52(1) |
| N(5) | 772(9) | 7946(6) | 8284(2) | 56(1) |
| N(6) | 1429(7) | 2512(5) | 3909(2) | 49(1) |
| N(7) | -2370(7) | 3389(5) | 4553(2) | 48(1) |
| N(8) | -4351(8) | 6711(6) | 4439(2) | 53(1) |
| N(9) | 3079(8) | 4538(6) | 3189(2) | 50(1) |
| N(10) | 8275(9) | 1678(6) | 1538(2) | 60(1) |
| O(1) | -1804(8) | 9164(6) | 9221(2) | 64(1) |
| O(2) | -1703(7) | 11274(6) | 8311(2) | 64(1) |
| O(3) | 358(12) | 14213(6) | 8737(3) | 109(2) |
| O(4) | 11221(7) | -1412(6) | 1605(2) | 61(1) |
| O(5) | 11836(9) | 976(7) | 769(2) | 82(1) |
| O(6) | 8669(12) | -4215(7) | 1300(3) | 102(2) |
| C(1) | 9497(9) | 7251(7) | 5530(2) | 51(1) |
| C(2) | 11653(8) | 4257(6) | 5572(2) | 42(1) |
| C(3) | 9901(8) | 3648(6) | 6010(2) | 43(1) |
| C(4) | 10849(10) | 1597(7) | 6121(2) | 58(1) |
| C(5) | 13010(11) | 1139(7) | 5754(3) | 63(1) |
| C(6) | 7881(8) | 5125(6) | 6218(2) | 42(1) |
| C(7) | 6126(13) | 3052(10) | 6998(3) | 83(2) |
| C(8) | 4441(10) | 6629(8) | 6943(2) | 59(1) |
| C(9) | 4822(11) | 7443(10) | 7560(3) | 78(2) |
| C(10) | 2036(10) | 8076(7) | 7672(2) | 54(1) |
| C(11) | 1787(10) | 6576(11) | 7200(3) | 74(2) |
| C(12) | 2049(10) | 10828(7) | 8938(2) | 56(1) |
| C(13) | 1179(11) | 12887(7) | 9205(2) | 64(1) |
| C(14) | 3201(16) | 13652(11) | 9476(3) | 91(2) |
| C(15) | -328(9) | 2187(6) | 4336(2) | 50(1) |
| C(16) | -2489(8) | 5197(6) | 4295(2) | 42(1) |
| C(17) | -735(8) | 5804(6) | 3855(2) | 44(1) |
| C(18) | -1668(10) | 7834(7) | 3739(3) | 60(1) |
| C(19) | -3824(11) | 8328(7) | 4100(3) | 64(1) |
| C(20) | 1289(8) | 4314(6) | 3650(2) | 41(1) |
| C(21) | 3017(14) | 6400(9) | 2870(3) | 81(2) |
| C(22) | 4650(9) | 2801(7) | 2908(2) | 54(1) |
| C(23) | 7350(9) | 2784(8) | 2665(2) | 58(1) |
| C(24) | 7023(9) | 1446(7) | 2151(2) | 55(1) |
| C(25) | 4269(11) | 2146(11) | 2263(3) | 76(2) |
| C(26) | 8625(11) | -1251(8) | 688(2) | 63(1) |
| C(27) | 7162(10) | -2701(8) | 1003(3) | 64(1) |
| C(28) | 5897(14) | -3640(12) | 540(4) | 93(2) |

### Example 3 - Alternate Preparation for (S)-2-Hydroxy-N-(cis-3-(methyl(7H-pyrrolo[2,3-d]pyrimidin-4-yl)amino)cyclobutyl)propane-1-sulfonamide (IA)

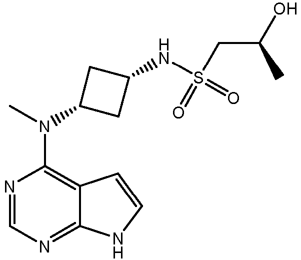

### Step 1. (S)-tert-Butyl((1-chloropropan-2-yl)oxy)dimethylsilane

To a solution of (S)-(+)-1-chloropropan-2-ol (1.0 g, 10.6 mmol) in N,N-dimethylformamide (20 mL) at 20 °C was added pyridine (1.0 g, 12.7 mmol) and tert-butyldimethylsilyl chloride (1.91 g, 12.7 mmol). After stirring for 20 °C, the reaction was diluted with water (40 mL). The resulting mixture was extracted with methyl *tert-butyl* ether (2 x 40 mL). The organic extracts were combined, washed with brine (50 mL), and dried (Na₂SO₄). The solvent was removed to give the title compound (2.2 g), which was used without further purification. ¹H NMR (400 MHz, CDCl₃) δ 3.96 (m, 1H), 3.42 (dd, *J =* 10.8, 5.9 Hz, 1H), 3.34 (dd, *J* = 10.7, 5.9 Hz, 1H), 1.23 (d, *J* = 6.1 Hz, 3H), 0.89 (s, 9H), 0.08 (d, *J* = 4.1 Hz, 6H).

### Step 2. (S)-S-(2-((tert-Butyldimethylsilyl)oxy)propyl) ethanethioate

To a solution of (S)-tert-butyl((1-chloropropan-2-yl)oxy)dimethylsilane (2.2 g, 10.5 mmol) in N,N-dimethylformamide (20 mL) at 20 °C was added potassium thioacetate (2.41 g, 21.1 mmol) and potassium iodide (17.5 mg, 0.10 mmol). The reaction was warmed to 80 °C and stirred for 20 hours. The reaction was cooled to room temperature, diluted with water (50 mL) and the mixture extracted with methyl *tert-butyl* ether (40 mL x 2). The organic extracts were collected, washed with brine (60 mL), dried (Na₂SO₄) and the solvent removed to give the title compound (2.40g), which was used without purification. ¹H NMR (400 MHz, CDCl₃) δ 3.91 (m, 1H), 3.00 - 2.91 (m, 2H), 2.33 (s, 3H), 1.18 (d, *J* = 6.1 Hz, 3H), 0.89 (s, 9H), 0.07 (d, *J* = 6.8 Hz, 6H).

### Step 3. (S)-2-Hydroxypropane-1-sulfonyl chloride

Cl_{2(g)} was passed through a solution of (*S*)-S-(2-((tert-butyldimethylsilyl)oxy)propyl) ethanethioate (0.5 g, 2.01 mmol) in dichloromethane (20 mL) and water (10 mL) at 0 °C for 5 min and the solution became yellow. The flow of Cl_{2(g)} was stopped and the reaction stirred at 0 °C for 0.5 hr. N_{2(g)} was then bubbled through the reaction to give a colorless solution. TLC indicated consumption of starting material and formation of a new spot. Water (20 mL) was added to the reaction mixture, which was then extracted with dichloromethane (40 mL). The organic extract was washed with 10% NaHCO₃ (50 mL), brine (50 mL) and dried (Na₂SO₄). The solvent was removed to afford the crude title compound (0.6 g) as a colorless oil, which was used in next step without purification.

### Step 4. (S)-2-Hydroxy-N-(cis-3-(methyl(7-tosyl-7H-pyrrolo[2,3-d]pyrimidin-4-yl)amino)cyclobutyl)propane-1-sulfonamide

To a 40 mL vial was added *cis*-N'-methyl-N'-(7-tosyl-7H-pyrrolo[2,3-d]pyrimidin-4-yl)cyclobutane-1,3-diamine (200 mg, 0.375 mmol), Na₂CO₃ (199 mg 1.88 mmol), tetrahydrofuran (7 mL) and water (2.5 mL). After 2 min, a solution of Example 1, Step 3 (292 mg) in tetrahydrofuran (2 mL) was added. The resulting suspension was heated to 50 °C and stirred for 18 hrs. The reaction was cooled to room temperature and water (20 mL) was added. The resulting mixture was extracted with ethyl acetate (20 mL x 2). The organic extracts were collected, dried (Na₂SO₄) and the solvent removed to give crude material, which was purified by chromatography (silica, EtOAc/PetEther, 20-100%) to give the title compound (30 mg). LC/MS *m*/*z* (M+H)⁺ = 494.3.

### Step 5. (S)-2-Hydroxy-N-(cis-3-(methyl(7H-pyrrolo[2,3-d]pyrimidin-4-yl)amino)cyclobutyl)propane-1-sulfonamide

To a solution of (*S*)-2-hydroxy-N-(*cis*-3-(methyl(7-tosyl-7H-pyrrolo[2,3-d]pyrimidin-4-yl)amino)cyclobutyl)propane-1-sulfonamide (45 mg, 0.091 mmol) in tetrahydrofuran:water (5 mL:1 mL) at 15 °C was added lithium hydroxide monohydrate (23.0 mg, 0.547 mmol). The reaction was then warmed to 60 °C and stirred for 16 hrs. Water (10 mL) was added to the reaction and the resulting mixture extracted with ethyl acetate (15 mL x 4). The organic extracts were combined, dried (Na₂SO₄) and the solvent removed to give the crude material (30 mg), which was then purified by RP-HPLC (Phenomenex Gemini C-18^{™}, 250 x 50 mm, 10µ, H₂O/CH₃CN+0.05% NH₄OH, 15-35% over 10 min) to give title compound (16 mg). ¹H NMR (400 MHz, DMSO) δ 11.63 (s, 1H), 8.09 (s, 1H), 7.41 (s, 1H), 7.14 (d, *J* = 3.6 Hz, 1H), 6.62 (d, *J* = 3.6 Hz, 1H), 4.89 (ddd, *J* = 17.2, 7.9, 5.9 Hz, 2H), 4.04 (m, 1H), 3.58 (m, 1H), 3.24 (s, 3H), 3.14 - 2.86 (m, 2H), 2.62 - 2.51 (m, 2H), 2.23 (m, 2H), 1.20 (d, *J* = 6.2 Hz, 3H);LC/MS *m*/*z* (M+H)⁺ = 340.1.; Chiral SFC^{™} (Chiralpak AD-3^{™}, 150 x 4.6mm, 3µ, 40% MeOH (0.05% DEA) in CO₂ isocratic 10 min, 2.5 mL/min, T = 35 °C) Rt = 5.54 min, 99% ee.

### Example 4 - Alternate Preparation for (R)-2-Hydroxy-N-(cis-3-(methyl(7H-pyrrolo[2,3-d]pyrimidin-4-yl)amino)cyclobutyl)propane-1-sulfonamide (IC)

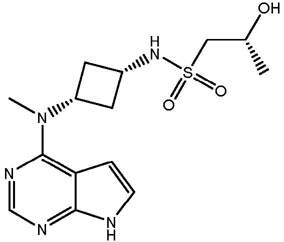

### Step 1. (R)-tert-Butyl((1-chloropropan-2-yl)oxy)dimethylsilane

To a solution of (*R*)-(-)-1-chloropropan-2-ol (2.0 g, 21.2 mmol, CAS: 19141-39-0) in N,N-dimethylformamide (40 mL) at 20 °C was added pyridine (2.0 g, 25.4 mmol) and tert-butyldimethylsilyl chloride (3.83 g, 25.4 mmol). After stirring for 20 hrs, the reaction was diluted with water (60 mL) and the resulting mixture was extracted with methyl *tert-*butyl ether (2 x 80 mL). The organic extracts were collected, washed with brine (100 mL) and dried (Na₂SO₄). The solvent was removed to give the title compound (4.60 g), which was used without further purification. ¹H NMR (400 MHz, CDCl₃) δ 3.96 (m, 1H), 3.42 (dd, *J* = 10.8, 5.9 Hz, 1H), 3.34 (dd, *J* = 10.7, 5.9 Hz, 1H), 1.23 (d, *J* = 6.1 Hz, 3H), 0.89 (s, 9H), 0.08 (d, *J* = 4.1 Hz, 6H).

### Step 2. (R)-S-(2-((tert-Butyldimethylsilyl)oxy)propyl) ethanethioate

To a solution (R)-tert-butyl((1-chloropropan-2-yl)oxy)dimethylsilane (4.60 g, 22.03 mmol) in N,N-dimethylformamide (40 mL) was added potassium thioacetate (5.03 g, 44.1 mmol) and potassium iodide (36.6 mg, 0.22 mmol). After 20 hrs at 80 °C, the reaction was cooled to room temperature, diluted with water (100 mL) and the resulting mixture extracted with methyl *tert-*butyl ether (100 mL x 2). The organic extracts were combined, dried (Na₂SO₄) and the solvent removed to give the title compound (5.0 g). ¹H NMR (400 MHz, CDCl₃) δ 3.90 (m, 1H), 3.00 - 2.91 (m, 2H), 2.33 (s, 3H), 1.18 (d, *J =* 6.1 Hz, 3H), 0.88 (s, 9H), 0.06 (d, *J* = 6.7 Hz, 6H).

### Step 3. (R)-2-((tert-Butyldimethylsilyl)oxy)propane-1-sulfonyl chloride

Cl_{2(g)} was passed through a solution of (R)-S-(2-((tert-butyldimethylsilyl)oxy)propyl) ethanethioate (0.5 g,, 2.01 mmol) in dichloromethane (20 mL) and water (10.0 mL) at 0 °C for 5 min and the solution became yellow. The flow of Cl_{2(g)} was stopped and the reaction stirred at 0 °C for 0.5 hr. N₂ was then bubbled through the reaction to give a colorless solution. TLC indicated consumption of starting material and formation of a new spot. Water (20 mL) was added to the reaction mixture, which was then extracted with dichloromethane (40 mL). The organic extract was washed with 10% NaHCO₃ (50 mL), brine (50 mL) and dried (Na₂SO₄). The solvent was removed to afford the title compound (0.4 g) as a colorless oil, which was used in next step without purification.

### Step 4. (R)-2-((tert-Butyldimethylsilyl)oxy)-N-((1s,3S)-3-(methyl(7-tosyl-7H-pyrrolo[2,3-d]pyrimidin-4-yl)amino)cyclobutyl)propane-1-sulfonamide

To a 40 mL vial was added *cis*-N¹-methyl-N¹-(7-tosyl-7H-pyrrolo[2,3-d]pyrimidin-4-yl)cyclobutane-1,3-diamine (150 mg, 0.281 mmol), Na₂CO₃ (149 mg 1.41 mmol), tetrahydrofuran (5 mL) and water (2.0 mL). After 2 min, a solution of Example 2, step 3 (292 mg, 0.75 mmol) in THF (2 mL) was added. The resulting suspension was heated to 50 °C and stirred for 20 hrs. The reaction was cooled to rt and water (20 mL) was added. The resulting mixture was extracted with EtOAc (20 mL x 2). The organic extracts were collected, dried (Na₂SO₄) and the solvent removed to give crude material, which was purified by chromatography (silica, EtOAc/PetEther, 20-100%) to give the title compound (60 mg) and material lacking TBS group (20 mg). LC/MS *m*/*z* (M+H)⁺ = 608.3 (pk 1); LC/MS *m*/*z* (M+H)⁺ = 494.3 (pk 2).

### Step 5. (R)-2-Hydroxy-N-(cis-3-(methyl(7-tosyl-7H-pyrrolo[2,3-d]pyrimidin-4-yl)amino)cyclobutyl)propane-1-sulfonamide

To a solution of (*R*)-2-((tert-butyldimethylsilyl)oxy)-N-(*cis*-3-(methyl(7-tosyl-7H-pyrrolo[2,3-d]pyrimidin-4-yl)amino)cyclobutyl)propane-1-sulfonamide (60 mg, 0.099 mmol) in dichloromethane (5 mL) at 0 °C was added trifluoroacetic acid (1.5 mL). After 16 hrs at 15 °C, the solvent was removed, and the residue dissolved in ethyl acetate (15 mL). The mixture was washed with saturated NaHCO₃(aq), dried (Na₂SO₄) and the solvent removed to give the title compound (50 mg), which was used in next step without purification. LC/MS m/z (M+H)⁺ = 494.3.

### Step 6. (R)-2-Hydroxy-N-(cis-3-(methyl(7H-pyrrolo[2,3-d]pyrimidin-4-yl)amino)cyclobutyl)propane-1-sulfonamide

To a solution of (*R*)-2-hydroxy-N-(*cis*-3-(methyl(7-tosyl-7H-pyrrolo[2,3-d]pyrimidin-4-yl)amino)cyclobutyl)propane-1-sulfonamide (50 mg, 0.10 mmol) in tetrahydrofuran:water (5 mL:1 mL) at 15 °C was added lithium hydroxide monohydrate (42.5 mg, 1.01 mmol). After 16 hrs at 65 °C, water (10 mL) was added and the mixture extracted with ethyl acetate (4 x 10 mL). The organic extracts were combined, dried (Na₂SO₄) and the solvent removed to give crude material, which was purified by prep-HPLC (Phenomenex Gemini C-18^{™}, 250 x 50 mm, 10µ, H₂O/CH₃CN+0.05% NH₄OH, 15-35% over 10 min) to give title compound (18 mg).¹H NMR (400 MHz, DMSO) δ 11.63 (s, 1H), 8.10 (s, 1H), 7.43 (d, *J* = 9.1 Hz, 1H), 7.15 (dd, *J* = 3.6, 2.4 Hz, 1H), 6.64 (dd, *J* = 3.6, 1.9 Hz, 1H), 4.97 - 4.83 (m, 2H), 4.12 - 3.98 (m, 1H), 3.59 (m, 1H), 3.25 (s, 3H), 3.13 - 2.92 (m, 2H), 2.58 (m, 2H), 2.24 (m, 2H), 1.21 (d, *J* = 6.2 Hz, 3H).; LC/MS m/z (M+H)⁺ = 340.0.; Chiral SFC (Chiralpak AD-3^{™}, 150 x 4.6mm, 3µ, 40% MeOH (0.05% DEA) in CO₂ isocratic 10 min, 2.5 mL/min, T = 35 °C) Rₜ = 6.75 min, 99% ee.

### Example 5 (for reference only) -- 3-Hydroxy-N-(cis-3-(methyl(7H-pyrrolo[2,3-d]pyrimidin-4-yl)amino)cyclobutyl)propane-1-sulfonamide (IB)

To a stirred mixture of cis-N-Methyl-N-7H-pyrrolo[2,3-d]pyrimidin-4-ylcyclobutane-1,3- diamine (190 mg) and potassium carbonate (240 mg) in solvent mixture of 1:1 tetrahydrofuran:water (5 mL each) at 10 °C was added methyl 3-(chlorosulfonyl)propanoate (250 mg). The mixture was warmed to room temperature and stirred for 10 min. The mixture was concentrated in vacuo. The residue was purified by column chromatography (MeOH/DCM 1:20) to afford methyl 3-(N-(cis-3-(methyl(7H-pyrrolo[2,3-d]pyrimidin-4-yl)amino)cyclobutyl)sulfamoyl)propanoate (140 mg) as a white solid. ¹H NMR (400 MHz, Methanol-*d₄*): 8.13 (s, 1H), 7.13 (d, 1H), 6.70 (d, 1H), 4.89 (m, 1H), 3.73 (s, 3H), 3.69 (m, 1H), 3.4 (m, 5H), 2.82 (m, 4H), 2.34 (m, 2H). MS m/z for C₁₅H₂₁N₅O₄S: 390.2 (M+Na)+. HPLC: Ultimate XB-C18 3pm 3.0*50mm column, retention time: 2.13 min, mobile phase: 0% acetonitrile (0.1% TFA) in water to 60% acetonitrile (0.1% TFA) in water, wavelength: 220 nm.

To a stirred solution of methyl 3-(N-(cis-3-(methyl(7H-pyrrolo[2,3-d]pyrimidin-4-yl)amino)cyclobutyl)sulfamoyl)propanoate (120 mg) in dry tetrahydrofuran (5 mL) was added lithium aluminum hydride (50 mg) at 0 °C under nitrogen. The reaction mixture was warmed to room temperature and was stirred for 0.5 hours. The mixture was quenched by the careful addition of methanol and purified directly by column chromatography (MeOH/DCM 1:10) to afford 3-hydroxy-N-(cis-3-(methyl(7H-pyrrolo[2,3-d]pyrimidin-4-yl)amino)cyclobutyl)propane-1-sulfonamide (50 mg) as a white solid. ¹H NMR (400 MHz, Methanol-*d₄*): 8.12 (s, 1H), 7.13 (d, 1H), 6.70 (d, 1H), 4.89 (m, 1H), 3.73 (m, 3H), 3.36 (s, 3H), 3.15 (m, 2H), 2.81 (m, 2H), 2.35 (m, 2H), 2.03 (m, 2H); MS m/z for C₁₄H₂₁N₅O₃S: 340.2 (M+H)+; HPLC: Ultimate XB-C18 3pm 3.0*50mm column, retention time: 1.85 min, mobile phase: 0% acetonitrile (0.1% TFA) in water to 60% acetonitrile (0.1% TFA) in water, wavelength: 220 nm.

### Example 6 (for reference only) -- Alternative Preparation of 3-Hydroxy-N-(cis-3-(methyl(7H-pyrrolo[2,3-d]pyrimidin-4-yl)amino)cyclobutyl)propane-1-sulfonamide

To a mixture of cis-N-methyl-N-{7-[(4-methylphenyl)sulfonyl]-7H-pyrrolo[2,3-d]-pyrimidin-4-yl}cyclobutane-1,3-diamine dihydrobromide (988 mg) in 50 mL dichloromethane was added triethylamine (562 mg) and ethyl 3-(chlorosulfonyl)propanoate (743 mg) at 0°C. The reaction mixture was warmed to 25°C and stirred for 2h. The reaction mixture was evaporated in vacuo and purified by silica gel chromatography (Biotage, 20 g silicon column, petroleum ether:ethyl acetate 1:0 to 0:1, ethyl acetate:methanol 20:1) to afford ethyl 3-(N-(cis-3-(methyl(7-tosyl-7H-pyrrolo[2,3-d]pyrimidin-4-yl)amino)cyclobutyl)sulfamoyl)propanoate. LCMS m/z for C₂₃H₂₉N₅O₆S₂: 535.9 (M+H)+.

To a solution of ethyl 3-(N-(cis-3-(methyl(7-tosyl-7H-pyrrolo[2,3-d]pyrimidin-4-yl)amino)cyclobutyl)sulfamoyl)propanoate (700 mg) in 50 mL tetrahydrofuran was added lithium aluminum hydride (74.4 mg) at 0 °C. The reaction mixture was allowed to warm to 25 °C for 15h. The reaction mixture was carefully quenched with 1 mL water, then filtered. The filtrate was evaporated in vacuo to afford the crude product 3-hydroxy-N-(cis-3-(methyl(7-tosyl-7H-pyrrolo[2,3-d]pyrimidin-4-yl)amino)cyclobutyl)propane-1-sulfonamide which was used directly without further purification. LCMS m/z for C₂₁H₂₇N₅O₅S₂: 494.0 (M+H)+.

To a solution of 3-hydroxy-N-(cis-3-(methyl(7-tosyl-7H-pyrrolo[2,3-d]pyrimidin-4-yl)amino)cyclobutyl)propane-1-sulfonamide (400 mg) in 20 mL ethanol and 10 mL water was added LiOH (97 mg) at 25 °C. The reaction mixture was heated 90 °C and stirred for 2 h. The reaction mixture was cooled and evaporated in vacuo to afford the crude product which was purified by prep-HPLC to give 181 mg of 3-hydroxy-N-(cis-3-(methyl(7H-pyrrolo[2,3-d]pyrimidin-4-yl)amino)cyclobutyl)propane-1-sulfonamide as a white solid. HPLC conditions: column: DuraShell^{™} 150*25mm*5um, water (0.05% ammonia hydroxide v/v)-acetonitrile, 1-41%B, gradient 10 min, hold time 1 min 100%B, flow rate 25mL/min; ¹H NMR (400 MHz, DMSO-*d₆*): 11.6 (s, 1H), 8.11 (s, 1H), 7.53 (s, 1H), 7.15 (d, 1H), 6.65 (d, 1H), 4.89 (m, 1H), 4.68 (s, 1H), 3.58 (m, 1H), 3.49 (m, 2H), 3.26 (s, 3H), 3.0 (m, 2H), 2.58 (m, 2H), 2.24 (m, 2H), 1.81 (m, 2H).

### Biological Evaluation

### JAK Caliper Enzyme Assay at 1mM ATP

Test article was solubilized in dimethyl sulfoxide (DMSO) to a stock concentration of 30 mM. An 11-point half log dilution series was created in DMSO with a top concentration of 600 µM. The test compound plate also contained positive control wells containing a known inhibitor to define 100% inhibition and negative control wells containing DMSO to define no inhibition. The compound plates were diluted 1 to 60 resulting in a top final assay compound concentration of 10 µM and a 2% DMSO concentration. Test article and assay controls were added to a 384-well plate. Reaction mixtures contained 20 mM HEPES, pH 7.4, 10 mM magnesium chloride, 0.01% bovine serum albumin (BSA), 0.0005% Tween 20, 1 mM ATP and 1 µM peptide substrate. The JAK1 and TYK2 assays contained 1 µM of the IRStide peptide (5FAM-KKSRGDYMTMQID) and the JAK2 and JAK3 assays contained 1 µM of the JAKtide peptide (FITC-KGGEEEEYFELVKK). The assays were initiated by the addition of 20 nM JAK1, 1 nM JAK2, 1 nM JAK3 or 1 nM TYK2 enzyme and were incubated at room temperature for three hours for JAK1, 60 minutes for JAK2, 75 minutes for JAK3 or 135 minutes for TYK2. Enzyme concentrations and incubation times were optimized for each new enzyme preps and were modified slightly over time to ensure 20%-30% phosphorylation. The assays were stopped with a final concentration of 10 mM EDTA, 0.1% Coating Reagent and 100 mM HEPES, pH=7.4. The assay plates were placed on a Caliper Life Science Lab Chip 3000 (LC3000) instrument, and each well was sampled using appropriate separation conditions to measure the unphosphorylated and phosphorylated peptide. Data are shown in Table 5.

**Table 5. JAK Caliper Enzyme Assay at 1mM ATP.**

| **Kinase** | **IC₅₀ nM** | | | |
|---|---|---|---|---|
| | **Abrocitinib** | **IA (M2)** | **IB (M1)** | **IC (M3)** |
| | **1 mM ATP** | **1 mM ATP** | **1 mM ATP** | **1 mM ATP** |
| JAK1 | 29.2 | 17.9 | 43.4 | 56.7 |
| JAK2 | 803 | 886 | 1140 | 2280 |
| JAK3 | >10,000 | >10,000 | >10,000 | >10000 |
| TYK2 | 1250 | 1210 | 3190 | 3420 |

### Cellular Potency Assays: Cytokine-induced Phosphorylation of STATs in Human Whole Blood, Human Keratinocytes and IFNy-primed THP-1 cells

Human whole blood was collected from healthy donors via vein puncture into Vacutainer collection tubes containing sodium heparin in accordance with Pfizer protocols (Protocol No. GOHW RDP-01) approved by the Shulman Institutional Review Board. Blood was warmed to 37°C prior to use. Human whole blood was aliquoted (90 µL/well) in 96-well, deep-well, V-bottom plates and treated with compounds (5 µL/well) at different concentrations up to 60 µM (0.2% DMSO final) at 37°C for 60 minutes. This was followed by a challenge with IFNα (5000 U/mL), IFNγ (100 ng/mL), IL-6 (50 ng/mL), IL-10 (30 ng/mL), IL-12 (30 ng/mL), IL-15 (30 ng/mL), IL-21 (50 ng/mL), IL-23 (25 ng/mL), IL-27 (1000 ng/mL), EPO (2U/mL), TSLP (50 ng/mL) or PBS and incubated for 15 minutes. Anti-cell surface antibodies were added 15 minutes prior to cytokine stimulation; anti-CD3-BV421 (0.5 µL/well) to IL-6-treated and TSLP-treated samples and anti-CD14-BV421 (0.5 µL/well) to IFNγ-treated samples. Samples were treated with warm 1x Lyse/Fix buffer (700 µL/well) to terminate activation and further incubated at 37°C for 20 minutes to lyse red blood cells. Plates were centrifuged at 300x g for 5 minutes, supernatant was aspirated, and cells were washed with 800 µL/well of staining buffer (D-PBS containing 0.1% FBS and 0.01% sodium azide). Washed cell pellets were resuspended in 350 µL/well of prechilled 90% methanol and incubated at 4°C for 30 minutes. After the removal of 90% methanol, cells were washed once with staining buffer. All samples were finally suspended in 150 µL/well of the desired anti-phospho-STAT antibodies at 1:150 dilution in staining buffer; anti-pSTAT1-AF647 in IFNγ treated samples; anti-pSTAT1-AF488 and anti-pSTAT3-AF647 in IL-6-treated samples, anti-pSTAT3-AF647 in IFNα, IL-10, IL-21, IL-23 and IL-27 treated samples, anti-pSTAT4-AF647 in IL-12 treated samples, anti-pSTAT5-AF-647 in EPO, IL-15 and TSLP-treated samples.

Human primary keratinocytes were cultured in DermaLife^{™} medium with supplement kit to expand cell population. Cell passages 2 to 5 were used in the assays. Cells were harvested at ~80% confluence, suspended in warm DermaLife^{™} medium, aliquoted (90 µL/well) in 96-well, deep-well, V-bottom plates, and incubated at 37°C for 30 minutes. Cells were then treated with compounds (0.0003 to 20 µM) for 60 minutes and followed by stimulation with IL-4 (2 ng/mL) or IL-13 (20 ng/mL) at 37°C for 15 minutes. For the IL-22 induced pSTAT3 assay, keratinocytes were seeded in 24-well plates and cultured for 18 hours. Cells were switched to DermaLife^{™} basal medium, treated with compounds (0.0003 to 20 µM) for 60 minutes, and then stimulated with IL-22 (100 ng/mL) for 30 minutes. Cells were detached by the treatment of 0.25% trypsin/EDTA. Stimulated keratinocytes were fixed by 2% paraformaldehyde and permeabilized by 90% methanol. Fixed and permeabilized cells were stained with AlexaFluor647^{™} labeled anti-pSTAT6 antibody (1 to 150 dilution) for IL-4 and IL-13-treated samples and AlexaFluor647 labeled anti-pSTAT3 (1 to 150 dilution) for IL-22 treated samples.

THP-1 cells were maintained in RPMI 1640 medium containing 10% FBS, 50 µM 2 mercaptoethanol, 50 U/mL penicillin, 50 µg/mL streptomycin and 2 mM L-glutamine. THP 1 cells were treated with IFNγ (20 ng/mL) for 18 hours. IFNγ primed THP-1 cells were resuspended in fresh RPMI 1640 medium, treated with compounds (0.0003 to 20 µM) for 60 minutes, followed by stimulation with IL-31(1 µg/mL) for additional 10 minutes. Cells were then fixed by 2% paraformaldehyde and permeabilized by 90% methanol. Fixed and permeabilized cells were stained with AlexaFluor647^{™} labeled anti-pSTAT3 antibody (1 to 150 dilution).

After overnight incubation at 4°C, anti-pSTAT stained samples were transferred into 96-well polypropylene U-bottom plates and flow cytometric analysis was performed on a LSR Fortessa^{™} equipped with a HTS plate loader. For human whole blood samples, lymphocyte population was gated for pSTAT histogram analysis for IFNα, IL-10, IL-12, IL-15, IL-21, IL-23, and IL-27-treated samples; CD14⁺ cells for IFNγ-treated samples; CD3⁺ cells for IL-6 and TSLP-treated samples; all events (entire populations) for EPO-treated samples. Entire populations of keratinocytes and THP-1 cells were gated for the IL-4, IL-13, IL-22 and IL-31 assays. Background fluorescence was defined using unstimulated cells and a gate was placed at the foot of the peak to include ~0.5% gated population. Histogram statistical analysis was performed using FACSDiva version 8.0. Relative fluorescence unit, which measures the level of pSTAT, was calculated by multiplying the percent positive population and its mean fluorescence. Inhibition curves and half maximal inhibitory concentration (IC₅₀) values were determined using Prism^{™} software (Version 8) or the Activity Base data analysis software (ID Business Solutions).

### Cellular Potency Against Cytokine-induced Phosphorylation of STATs

Compounds of formula **IA** and **IB,** prepared in accord with Examples 1 and 3 and Examples 5 and 6, respectively, are formed *in vivo* in human metabolism upon ingestion of abrocitinib, and demonstrate activity similar to abrocitinib, a potent JAK1 inhibitor, against IL-4, IL-13, IL-22, IL 31 and TSLP induced phosphorylation of STATs in human keratinocytes, interferon-γ primed THP-1 cells or human whole blood. Gooderham M.J., et al., JAMA Dermatology, 155(12), 1371-1379 (October 2019). Notably, like abrocitinib, these compounds are all more potent against cytokines which transduce their signals via JAK1-dependent pathways versus those which transduce their signals via JAK1-independent pathways, and therefore likely contribute to the pharmacological activity mainly via effects on JAK1. A comparison of the cellular potency for abrocitinib and the compounds of formula **IA** and **IB** across similar signaling pathways is shown in Table 6.

**Table 6. Cellular potency (IC₅₀, nM) of Compounds IA, IB and IC: STAT phosphorylation in IL-4, IL-13, IL-22, IL-31 and TSLP treated keratinocytes, IFNγ primed THP-1 cells or whole blood.**

| | | **Abrocitinib** | **IA (M2)** | **IB (M1)** | **IC (M3)** |
|---|---|---|---|---|---|
| IFNg induced pSTAT1 | JAK1/JAK2 | 1690 | 2160 | 1950 | 4250 |
| IL-4 induced pSTAT6 | JAK1 /JAK2 | 77.0 | 134 | 433 | |
| IL-6 induced pSTAT1 | JAK1/JAK2 | 343 | 136 | 171 | 333 |
| IL-13 induced pSTAT6 | JAK1/JAK2 | 81.9 | 84.1 | 236 | |
| IL-27 induced pSTAT3 | JAK1/JAK2 | 228 | 234 | 382 | 572 |
| IL-31 induced pSTAT3 | JAK1/JAK2 | 40.0 | 56.0 | 79.6 | |
| TSLP induced pSTAT5 | JAK1/JAK2 | 1020 | 271 | 785 | |
| IL-15 induced pSTAT5 | JAK1/JAK3 | 537 | 353 | 558 | 800 |
| IL-21 induced pSTAT3 | JAK1/JAK3 | 516 | 487 | 844 | 1050 |
| IL-22 induced pSTAT3 | JAK1/TYK2 | 420 | 198 | 703 | |
| IL-10 induced pSTAT3 | JAK1/TYK2 | 3700 | 233 | 675 | 488 |
| IFNa induced pSTAT3 | JAK1/TYK2 | 174 | 90.5 | 296 | 225 |
| EPO induced pSTAT5 | JAK2/JAK2 | 7180 | 9470 | 9750 | 18100 |
| IL-23 induced pSTAT3 | JAK2/TYK2 | >16500 | 6210 | 26200 | 13800 |

## Claims

1. A compound of formula IA, having the structure: 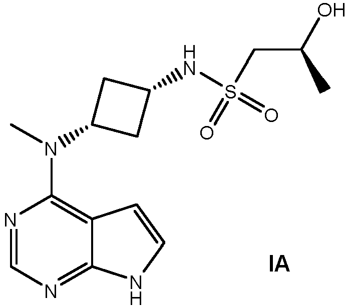 or a pharmaceutically acceptable salt thereof.

2. A compound of formula IC, having the structure: 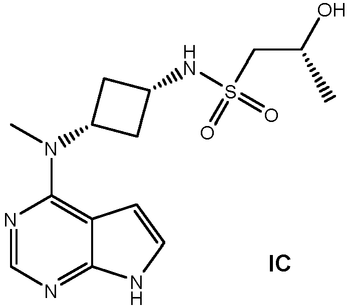 or a pharmaceutically acceptable salt thereof.

3. The compound or a pharmaceutically acceptable salt thereof according to claim 1 or claim 2 in an isolated form.

4. A pharmaceutical composition comprising a compound of claim 1 or claim 2, or a pharmaceutically acceptable salt thereof, and a pharmaceutically acceptable excipient.

5. A compound of claim 1 or claim 2, or a pharmaceutically acceptable salt thereof, for use in a method of treating or preventing a disease or condition selected from inflammation, autoimmune disease, neuroinflammation, arthritis, rheumatoid arthritis, spondyloarthropathies, systemic lupus erythematous, lupus nephritis, osteoarthritis, gouty arthritis, pain, fever, pulmonary sarcoidosis, silicosis, cardiovascular disease, atherosclerosis, myocardial infarction, thrombosis, congestive heart failure and cardiac reperfusion injury, cardiomyopathy, stroke, ischemia, reperfusion injury, brain edema, brain trauma, neurodegeneration, liver disease, inflammatory bowel disease, Crohn's disease, ulcerative colitis, nephritis, retinitis, retinopathy, macular degeneration, glaucoma, diabetes (type 1 and type 2), diabetic neuropathy, viral and bacterial infection, myalgia, endotoxic shock, toxic shock syndrome, osteoporosis, multiple sclerosis, endometriosis, menstrual cramps, vaginitis, candidiasis, cancer, fibrosis, obesity, muscular dystrophy, polymyositis, dermatomyositis, autoimmune hepatitis, primary biliary cirrhosis, primary sclerosing cholangitis, vitiligo, Alzheimer's disease, skin flushing, eczema, psoriasis, atopic dermatitis, sunburn, keloid, hypertrophic scar, rheumatic diseases, urticaria, discoid lupus, cutaneous lupus, central nervous system lupus, psoriatic arthritis, asthma, allergic asthma, type I interferonopathies including Aicardi-Goutières syndrome and other mendelian diseases of overexpression of type I interferon, primary progressive multiple sclerosis, relapsing remitting multiple sclerosis, non-alcoholic fatty liver disease, non-alcoholic steatohepatitis, scleroderma, alopecia areata, scarring alopecia, prurigo, prurigo nodularis, CPUO, lichen diseases, lichen planus, Steven's Johnson's syndrome, spondylopathy, myositis, vasculitis, pemphigus, lupus, major depression disorder, allergy, dry eye syndrome, transplant rejection, cancer, septic shock, cardiopulmonary dysfunction, acute respiratory disease, ankylosing spondylitis, cachexia, chronic graft-versus-host disease, acute graft-versus-host disease, Celiac Sprue, idiopathic thrombocytopenic thrombotic purpura, thrombotic thrombocytopenic purpura, myasthenia gravis, Sjogren's syndrome, epidermal hyperplasia, cartilage inflammation, bone degradation, juvenile arthritis, juvenile rheumatoid arthritis, pauciarticular juvenile rheumatoid arthritis, polyarticular juvenile rheumatoid arthritis, systemic onset juvenile rheumatoid arthritis, juvenile ankylosing spondylitis, juvenile enteropathic arthritis, juvenile Reter's Syndrome, SEA Syndrome, juvenile dermatomyositis, juvenile psoriatic arthritis, juvenile scleroderma, juvenile systemic lupus erythematosus, juvenile vasculitis, pauciarticular rheumatoid arthritis, polyarticular rheumatoid arthritis, systemic onset rheumatoid arthritis, enteropathic arthritis, reactive arthritis, Reter's Syndrome, myolitis, polymyolitis, dermatomyolitis, polyarteritis nodossa, Wegener's granulomatosis, arteritis, polymyalgia rheumatica, sarcoidosis, sclerosis, primary biliary sclerosis, sclerosing cholangitis, dermatitis, Still's disease, chronic obstructive pulmonary disease, Guillain-Barre disease, Graves' disease, Addison's disease, Raynaud's phenomenon, psoriatic epidermal hyperplasia, plaque psoriasis, guttate psoriasis, inverse psoriasis, pustular psoriasis, erythrodermic psoriasis, an immune disorder associated with or arising from activity of pathogenic lymphocytes, noninfectious uveitis, Behcet's disease and Vogt-Koyanagi-Harada syndrome, the method comprising administering to a subject in need thereof a therapeutically effective amount of a compound of claim 1 or claim 2, or a pharmaceutically acceptable salt thereof.

6. A compound, or a pharmaceutically acceptable salt thereof, for use in a method of treating or preventing a disease or condition, as claimed in claim 5, wherein the disease or condition is atopic dermatitis.

7. A compound, or a pharmaceutically acceptable salt thereof, for use in a method of treating or preventing a disease or condition, as claimed in claim 5, wherein the disease or condition is hand eczema.

8. A compound, or a pharmaceutically acceptable salt thereof, for use in a method of treating or preventing a disease or condition, as claimed in claim 5, wherein the disease or condition is cutaneous lupus.

## Patentansprüche

1. Verbindung der Formel IA mit der Struktur: 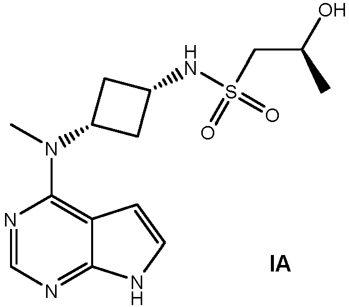 oder ein pharmazeutisch annehmbares Salz davon.

2. Verbindung der Formel IC mit der Struktur: 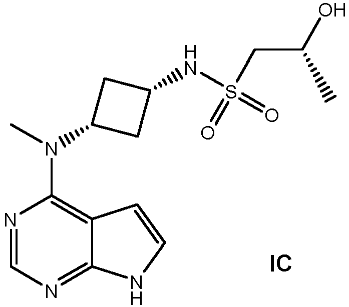 oder ein pharmazeutisch annehmbares Salz davon.

3. Verbindung oder ein pharmazeutisch annehmbares Salz davon nach Anspruch 1 oder Anspruch 2 in einer isolierten Form.

4. Pharmazeutische Zusammensetzung, umfassend eine Verbindung nach Anspruch 1 oder Anspruch 2 oder ein pharmazeutisch annehmbares Salz davon und einen pharmazeutisch annehmbaren Hilfsstoff.

5. Verbindung nach Anspruch 1 oder Anspruch 2 oder ein pharmazeutisch annehmbares Salz davon zur Verwendung in einem Verfahren zur Behandlung oder Prävention einer Erkrankung oder eines Zustands, ausgewählt aus Entzündung, Autoimmunerkrankung, Neuroinflammation, Arthritis, rheumatoider Arthritis, Spondyloarthropathien, systemischem Lupus erythematodes, Lupusnephritis, Osteoarthritis, Gichtarthritis, Schmerz, Fieber, pulmonaler Sarkoidose, Silikose, kardiovaskulärer Erkrankung, Atherosklerose, Myokardinfarkt, Thrombose, kongestiver Herzinsuffizienz und kardialer Reperfusionsverletzung, Kardiomyopathie, Schlaganfall, Ischämie, Reperfusionsverletzung, Hirnödem, Hirntrauma, Neurodegeneration, Lebererkrankung, entzündlicher Darmerkrankung, Morbus Crohn, Colitis ulcerosa, Nephritis, Retinitis, Retinopathie, Makuladegeneration, Glaukom, Diabetes (Typ 1 und Typ 2), diabetischer Neuropathie, viraler und bakterieller Infektion, Myalgie, endotoxischem Schock, toxischem Schocksyndrom, Osteoporose, multipler Sklerose, Endometriose, Menstruationskrämpfen, Vaginitis, Candidiasis, Krebs, Fibrose, Adipositas, Muskeldystrophie, Polymyositis, Dermatomyositis, Autoimmunhepatitis, primärer biliärer Zirrhose, primärer sklerosierender Cholangitis, Vitiligo, Alzheimer-Krankheit, Hautrötung, Ekzem, Psoriasis, atopischer Dermatitis, Sonnenbrand, Keloid, hypertropher Narbe, rheumatischen Erkrankungen, Urtikaria, diskoidem Lupus, kutanem Lupus, Lupus des zentralen Nervensystems, psoriatischer Arthritis, Asthma, allergischem Asthma, Typ-I-Interferonopathien, einschließlich Aicardi-Goutières-Syndrom und anderen mendelischen Erkrankungen der Überexpression von Typ-I-Interferon, primärer progressiver multipler Sklerose, rezidivierender remittierender multipler Sklerose, nichtalkoholischer Fettlebererkrankung, nichtalkoholischer Steatohepatitis, Sklerodermie, Alopecia areata, Narbenalopecia, Prurigo, Prurigo nodularis, CPUO, Lichen-Erkrankungen, Lichen planus, Stevens-Johnson-Syndrom, Spondylopathie, Myositis, Vaskulitis, Pemphigus, Lupus, schwerer Depressionsstörung, Allergie, Syndrom des trockenen Auges, Transplantatabstoßung, Krebs, septischem Schock, kardiopulmonaler Dysfunktion, akuter Atemwegserkrankung, Spondylitis ankylosans, Kachexie, chronischer Graft-versus-Host-Erkrankung, akuter Graft-versus-Host-Erkrankung, Zöliakie, idiopathischer thrombozytopenischer thrombotischer Purpura, thrombotischer thrombozytopenischer Purpura, Myasthenia gravis, Sjögren-Syndrom, epidermaler Hyperplasie, Knorpelentzündung, Knochenabbau, juveniler Arthritis, juveniler rheumatoider Arthritis, pauciartikulärer juveniler rheumatoider Arthritis, polyartikulärer juveniler rheumatoider Arthritis, systemisch einsetzender juveniler rheumatoider Arthritis, juveniler Spondylitis ankylosans, juveniler enteropathischer Arthritis, juvenilem Reter-Syndrom, SEA-Syndrom, juveniler Dermatomyositis, juveniler psoriatischer Arthritis, juvenilem Sklerodermie, juvenilem systemischem Lupus erythematodes, juveniler Vaskulitis, pauciartikulärer rheumatoider Arthritis, polyartikulärer rheumatoider Arthritis, systemisch einsetzender rheumatoider Arthritis, enteropathischer Arthritis, reaktiver Arthritis, Reter-Syndrom, Myolitis, Polymyolitis, Dermatomyolitis, Polyarteriitis nodossa, Wegener-Granulomatose, Arteriitis, Polymyalgia rheumatica, Sarkoidose, Sklerose, primärer biliärer Sklerose, sklerosierender Cholangitis, Dermatitis, Morbus Still, chronisch obstruktiver Lungenerkrankung, Guillain-Barre-Krankheit, Morbus Basedow, Morbus Addison, Raynaud-Phänomen, psoriatischer epidermaler Hyperplasie, Plaque-Psoriasis, Guttat-Psoriasis, inverser Psoriasis, pustularer Psoriasis, erythrodermischer Psoriasis, einer Immunstörung, die mit Aktivität pathogener Lymphozyten assoziiert ist oder daraus entsteht, nichtinfektiöser Uveitis, Morbus Behcet und Vogt-Koyanagi-Harada-Syndrom, wobei das Verfahren das Verabreichen einer therapeutisch wirksamen Menge einer Verbindung nach Anspruch 1 oder Anspruch 2 oder eines pharmazeutisch annehmbaren Salzes davon an ein Subjekt, das dessen bedarf, umfasst.

6. Verbindung oder ein pharmazeutisch annehmbares Salz davon zur Verwendung in einem Verfahren zur Behandlung oder Prävention einer Erkrankung oder eines Zustands nach Anspruch 5, wobei die Erkrankung oder der Zustand atopische Dermatitis ist.

7. Verbindung oder ein pharmazeutisch annehmbares Salz davon zur Verwendung in einem Verfahren zur Behandlung oder Prävention einer Erkrankung oder eines Zustands nach Anspruch 5, wobei die Erkrankung oder der Zustand Handekzem ist.

8. Verbindung oder ein pharmazeutisch annehmbares Salz davon zur Verwendung in einem Verfahren zur Behandlung oder Prävention einer Erkrankung oder eines Zustands nach Anspruch 5, wobei die Erkrankung oder der Zustand kutaner Lupus ist.

## Revendications

1. Composé de formule IA, ayant la structure : 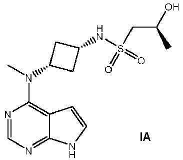 ou un sel pharmaceutiquement acceptable de celui-ci.

2. Composé de formule IC, ayant la structure : 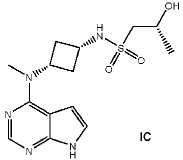 ou un sel pharmaceutiquement acceptable de celui-ci.

3. Composé ou sel pharmaceutiquement acceptable de celui-ci selon la revendication 1 ou la revendication 2 sous une forme isolée.

4. Composition pharmaceutique comprenant un composé selon la revendication 1 ou la revendication 2, ou un sel pharmaceutiquement acceptable de celui-ci, et un excipient pharmaceutiquement acceptable.

5. Composé selon la revendication 1 ou la revendication 2, ou sel pharmaceutiquement acceptable de celui-ci, pour une utilisation dans un procédé de traitement ou de prévention d'une maladie ou d'une affection sélectionnée parmi une inflammation, une maladie auto-immune, une neuroinflammation, l'arthrite, la polyarthrite rhumatoïde, les spondyloarthropathies, le lupus érythémateux disséminé, la néphrite lupique, l'arthrose, l'arthrite goutteuse, une douleur, la fièvre, la sarcoïdose pulmonaire, la silicose, une maladie cardiovasculaire, l'athérosclérose, un infarctus du myocarde, une thrombose, une insuffisance cardiaque congestive et une lésion de reperfusion cardiaque, une cardiomyopathie, un accident vasculaire cérébral, une ischémie, une lésion de reperfusion, un œdème cérébral, un traumatisme cérébral, une neurodégénérescence, une maladie hépatique, une maladie inflammatoire de l'intestin, la maladie de Crohn, la rectocolite hémorragique, une néphrite, une rétinite, une rétinopathie, une dégénérescence maculaire, un glaucome, un diabète (type 1 et type 2), une neuropathie diabétique, une infection virale et bactérienne, une myalgie, un choc endotoxique, le syndrome de choc toxique, l'ostéoporose, la sclérose en plaques, l'endométriose, les crampes menstruelles, une vaginite, une candidose, un cancer, une fibrose, l'obésité, une dystrophie musculaire, une polymyosite, une dermatomyosite, une hépatite auto-immune, une cirrhose biliaire primaire, une cholangite sclérosante primaire, un vitiligo, la maladie d'Alzheimer, une rougeur cutanée, un eczéma, un psoriasis, une dermatite atopique, un érhytème solaire, un chéloïde, une cicatrice hypertrophique, les maladies rhumatismales, l'urticaire, un lupus discoïde, un lupus cutané, un lupus du système nerveux central, une arthrite psoriasique, l'asthme, l'asthme allergique, les interféronopathies de type I notamment le syndrome d'Aicardi-Goutières et d'autres maladies mendéliennes de surexpression de l'interféron de type I, la sclérose en plaques progressive primaire, la sclérose en plaques rémittente-récurrente, une hépatopathie grasse non alcoolique, une stéatohépatite non alcoolique, une sclérodermie, une alopécie en aires, une alopécie cicatricielle, un prurigo, un prurigo nodularis, un CPUO, les maladies à lichen, un lichen plan, le syndrome de Steven-Johnson, une spondylopathie, une myosite, une vascularite, un pemphigus, un lupus, un trouble dépressif majeur, une allergie, le syndrome de l'œil sec, un rejet de greffe, un cancer, un choc septique, un dysfonctionnement cardiopulmonaire, une maladie respiratoire aiguë, une spondylarthrite ankylosante, une cachexie, une réaction chronique de greffe contre hôte, une réaction aiguë de greffe contre hôte, une maldie cœliaque, un purpura thrombocipénique thrombotique idiopathique, un purpura thrombocipénique thrombotique, une myasthénie grave, le syndrome de Sjögren, une hyperplasie épidermique, une inflammation du cartilage, une dégradation osseuse, l'arthrite juvénile, la polyarthrite rhumatoïde juvénile, la polyarthrite rhumatoïde juvénile pauci-articulaire, la polyarthrite rhumatoïde juvénile polyarticulaire, la polyarthrite rhumatoïde juvénile à début systémique, la spondylarthrite ankylosante juvénile, l'arthrite entéropathique juvénile, le syndrome de Reter juvénile, le syndrome SEA, la dermatomyosite juvénile, l'arthrite psoriasique juvénile, la sclérodermie juvénile, le lupus érythémateux disséminé juvénile, la vascularite juvénile, la polyarthrite rhumatoïde pauci-articulaire, la polyarthrite rhumatoïde polyarticulaire, la polyarthrite rhumatoïde à début systémique, l'arthrite entéropathique, l'arthrite réactive, le syndrome de Reter, la myosite, la polymyosite, la dermatomyosite, la périartérite noueuse, la granulomatose de Wegener, l'artérite, la polymyalgie rhumatismale, la sarcoïdose, une sclérose, la sclérose biliaire primaire, la cholangite sclérosante, la dermatite, la maladie de Still, la bronchopneumopathie chronique obstructive, la maladie de Guillain-Barré, la maladie de Graves, la maladie d'Addison, le phénomène de Raynaud, l'hyperplasie épidermique psoriasique, le psoriasis en plaques, le psoriasis en gouttes, le psoriasis inversé, le psoriasis pustuleux, le psoriasis érythrodermique, une maladie immunitaire associée à ou découlant de l'activité de lymphocytes pathogènes, une uvéite non infectieuse, la maladie de Behcet et le syndrome de Vogt-Koyanagi-Harada, le procédé comprenant l'administration à un sujet qui en a besoin d'une quantité thérapeutiquement efficace d'un composé selon la revendication°1 ou la revendication°2, ou d'un sel pharmaceutiquement acceptable de celui-ci.

6. Composé, ou sel pharmaceutiquement acceptable de celui-ci, pour une utilisation dans un procédé de traitement ou de prévention d'une maladie ou d'une affection, selon la revendication 5, dans lequel la maladie ou l'affection est une dermatite atopique.

7. Composé, ou sel pharmaceutiquement acceptable de celui-ci, pour une utilisation dans un procédé de traitement ou de prévention d'une maladie ou d'une affection, selon la revendication 5, dans lequel la maladie ou l'affection est l'eczéma de la main.

8. Composé, ou sel pharmaceutiquement acceptable de celui-ci, pour une utilisation dans un procédé de traitement ou de prévention d'une maladie ou d'une affection, selon la revendication 5, dans lequel la maladie ou l'affection est un lupus cutané.
